# EUROPEAN PATENT SPECIFICATION

(11) **EP 1 283 878 B1**
(45) Date of publication and mention of the grant of the patent: **27.07.2005**
(21) Application number: 01960237.4
(22) Date of filing: 22.05.2001
(51) Int. Cl.: C12N 15/10, C12N 15/62, C12N 5/10, C12N 1/19, C12N 1/15, C07K 14/715, G01N 33/566

(54) **RECEPTOR-BASED INTERACTION TRAP**
AUF REZEPTORGRUNDLAGE ARBEITENDE SCREENING-VERFAHREN FÜR PROTEIN WECHSELWIRKUNGEN
PIEGE A INTERACTION BASE SUR DES RECEPTEURS

(30) Priority: 22.05.2000 EP 00201771
(43) Date of publication of application: 19.02.2003
(73) Proprietor: Vlaams Interuniversitair Instituut voor Biotechnologie vzw., 9052 Zwijnaarde (BE)
(72) Inventor: EYCKERMAN, Sven, B-9000 Gent (BE); VAN OSTADE, Xaveer, B-9160 Lokeren (BE); VANDEKERCKHOVE, Joel, B-8210 Loppem (BE); VERHEE, Annick, B-8810 Lichtervelde (BE); TAVERNIER, Jan, B-9860 Balegem (BE)
(74) Representative: De Clercq, Ann
(86) International application number: PCT/EP2001/005916
(87) International publication number: WO 2001/090188

(56) References cited:
- EP-A- 0 646 644
- WO-A-00/06722
- WO-A-00/07038
- WO-A-97/31113
- WO-A-98/02542
- WO-A-98/34120
- WO-A-98/44350
- WO-A-99/03974
- US-A- 5 776 689
- OSBORNE M A ET AL: "THE YEAST TRIBRID SYSTEM - GENETIC DETECTION OF TRANS-PHOSPHORYLATED ITAM-SH2-INTERACTIONS" BIO/TECHNOLOGY,US,NATURE PUBLISHING CO. NEW YORK, vol. 13, 1 December 1995 (1995-12-01), pages 1474-1478, XP002033515 ISSN: 0733-222X
- FIELDS S ET AL: "THE TWO-HYBRID SYSTEM: AN ASSAY FOR PROTEIN-PROTEIN INTERACTIONS" TRENDS IN GENETICS,NL,ELSEVIER SCIENCE PUBLISHERS B.V. AMSTERDAM, vol. 10, no. 8, 1 August 1994 (1994-08-01), pages 286-292, XP000647708 ISSN: 0168-9525
- COLAS P ET AL: "The impact of two-hybrid and related methods on biotechnology" TRENDS IN BIOTECHNOLOGY,GB,ELSEVIER PUBLICATIONS, CAMBRIDGE, vol. 16, no. 8, 1 December 1998 (1998-12-01), pages 355-363, XP004131483 ISSN: 0167-7799
- EYCKERMAN S ET AL: "Analysis of Tyr to Phe and fa/fa leptin receptor mutations in the PC12 cell line" EUR CYTOKINE NETW, vol. 10, no. 4, December 1999 (1999-12), pages 549-556, XP000979093 cited in the application
- BANKS A ET AL: "Activation of downstream signals by the long form of the leptin receptor" JOURNAL OF BIOLOGICAL CHEMISTRY., vol. 275, no. 19, 12 May 2000 (2000-05-12), pages 14563-14572, XP002160348
- EYCKERMAN S ET AL: "Design and application of a cytokine-receptor-based interaction trap" NAT CELL BIOL, vol. 3, no. 12, December 2001 (2001-12), pages 1114-1119, XP001064660

### SEQUENCE LISTING

<110> Vlaams Interuniversitair Instituut voor Biotechnol
<120> RECEPTOR-BASED INTERACTION TRAP
<130> JT/MAG2/V055
<140>
   <141>
<150> 00201771.3
   <151> 2000-05-22
<160> 47
<170> PatentIn Ver. 2.1
<210> 1
   <211> 41
   <212> DNA
   <213> Artificial Sequence
<220>
   <223> Description of Artificial Sequence: forward
   primer: MBU-O-157; Y985F mutagenesis in mLepR
<400> 1
   gagacaaccc tcagttaaat ttgcaactct ggtcagcaac g                41
<210> 2
   <211> 41
   <212> DNA
   <213> Artificial Sequence
<220>
   <223> Description of Artificial Sequence: reverse
   primer: MBU-O-158; Y985F mutagenesis in mLepR
<400> 2
   cgttgctgac cagagttgca aatttaactg agggttgtct c 41
<210> 3
   <211> 41
   <212> DNA
   <213> Artificial Sequence
<220>
   <223> Description of Artificial Sequence: forward
   primer: MBU-O-159; Y1077F mutagenesis in mLepR
<400> 3
   gggagaagtc tgtctgtttt ctaggggtca cctccgtcaa c 41
<210> 4
   <211> 41
   <212> DNA
   <213> Artificial Sequence
<220>
   <223> Description of Artificial Sequence: reverse
   primer: MBU-O-160; Y1077F mutagenesis in mLepR
<400> 4
   gttgacggag gtgaccccta gaaaacagac agacttctcc c 41
<210> 5
   <211> 43
   <212> DNA
   <213> Artificial Sequence
<220>
   <223> Description of Artificial Sequence: forward
   primer: MBU-O-161; Y1138F mutagenesis in mLepR
<400> 5
   ctggtgagaa ctttgtacct tttatgcccc aatttcaaac ctg 43
<210> 6
   <211> 43
   <212> DNA
   <213> Artificial Sequence
<220>
   <223> Description of Artificial Sequence: reverse
   primer: MBU-O-162: Y1138F mutagenesis in mLepR
<400> 6
   caggtttgaa attggggcat aaaaggtaca aagttctcac cag 43
<210> 7
   <211> 30
   <212> DNA
   <213> Artificial Sequence
<220>
   <223> Description of Artificial Sequence: forward
   primer: MBU-O-167; hEpoR primer
<400> 7
   cggggtacca tggaccacct cggggcgtcc 30
<210> 8
   <211> 22
   <212> DNA
   <213> Artificial Sequence
<220>
   <223> Description of Artificial Sequence: forward
   primer: MBU-O-222; rPAP1 promoter primer
<400> 8
   ctgcagattt tccagttagt ca 22
<210> 9
   <211> 20
   <212> DNA
   <213> Artificial Sequence
<220>
   <223> Description of Artificial Sequence: reverse
   primer: MBU-O-223; rPAP1 promoter primer
<400> 9
   tggatggttt gtgaggacag 20
<210> 10
   <211> 32
   <212> DNA
   <213> Artificial Sequence
<220>
   <223> Description of Artificial Sequence: reverse
   primer: MBU-O-308; hEpoR primer
<400> 10
   cccttaatta agtccaggtc gctaggcgtc ag 32
<210> 11
   <211> 35
   <212> DNA
   <213> Artificial Sequence
<220>
   <223> Description of Artificial Sequence: reverse
   primer: MBU-O-493; hgp130 primer
<400> 11
   gcgaattccg aaccgccctg aggcatgtag ccgcc 35
<210> 12
   <211> 32
   <212> DNA
   <213> Artificial Sequence
<220>
   <223> Description of Artificial Sequence: forward
   primer: MBU-O-445; SV40LargeT primer
<400> 12
   gcgaattcga agcagaggaa actaaacaag tg 32
<210> 13
   <211> 55
   <212> DNA
   <213> Artificial Sequence
<220>
   <223> Description of Artificial Sequence: reverse
   primer: MBU-O-446; SV40LargeT primer
<400> 13
   cgtctagagc ggccgcagat ctcgagtcgc gattatgttt caggttcagg gggag 55
<210> 14
   <211> 31
   <212> DNA
   <213> Artificial Sequence
<220>
   <223> Description of Artificial Sequence: forward
   primer: MBU-O-947; mLepR intracellular and transmembrane fragment
<400> 14
   gcttaattaa cgggctgtat gtcattgtac c 31
<210> 15
   <211> 63
   <212> DNA
   <213> Artificial Sequence
<220>
   <223> Description of Artificial Sequence: reverse
   primer: MBU-O-448; mLepR intracellular and transmembrane fragment
<400> 15
   cgtctagatt agcggccgct tactagtgag ctcgtcgacc cacccacagt taagtcacac 60
   atc 63
<210> 16
   <211> 27
   <212> DNA
   <213> Artificial Sequence
<220>
   <223> Description of Artificial Sequence: forward
   primer: MBU-O-450; mp53 primer
<400> 16
   gtgtcgacgg tcaccgagac ccctggg 27
<210> 17
   <211> 28
   <212> DNA
   <213> Artificial Sequence
<220>
   <223> Description of Artificial Sequence: reverse
   primer: MBU-O-451; mp53 primer
<400> 17
   gctctagatc attgcggggg agaggcgc 28
<210> 18
   <211> 27
   <212> DNA
   <213> Artificial Sequence
<220>
   <223> Description of Artificial Sequence: forward
   primer: MBU-O-967; hGRB2 primer
<400> 18
   ggaattcatg gaagccatcg ccaaata 27
<210> 19
   <211> 28
   <212> DNA
   <213> Artificial Sequence
<220>
   <223> Description of Artificial Sequence: reverse
   primer: MBU-O-468; hGRB2 primer
<400> 19
   gctctagatt agacgttccg gttcacgg 28
<210> 20
   <211> 27
   <212> DNA
   <213> Artificial Sequence
<220>
   <223> Description of Artificial Sequence: forward
   primer: MBU-O-469; hGRB2 SH2 primer
<400> 20
   ggaattctgg ttttttggca aaatccc 27
<210> 21
   <211> 28
   <212> DNA
   <213> Artificial Sequence
<220>
   <223> Description of Artificial Sequence: reverse
   primer: MBU-O-470; hGRB2 SH2 primer
<400> 21
   gctctagatt acggctgctg tggcacct 28
<210> 22
   <211> 59
   <212> DNA
   <213> Artificial Sequence
<220>
   <223> Description of Artificial Sequence: forward
   primer: MBU-O-515; mutagenesis IRS1 primer
<400> 22
   ggtgagaact ttgtaagccc gggtgaatat gtcaatattg aattccaatt tcaaacctg 59
<210> 23
   <211> 59
   <212> DNA
   <213> Artificial Sequence
<220>
   <223> Description of Artificial Sequence: reverse
   primer: MBU-O-516; mutagenesis IRS1 primer
<400> 23
   caggtttgaa attggaattc aatattgaca tattcacccg ggcttacaaa gttctcacc 59
<210> 24
   <211> 38
   <212> DNA
   <213> Artificial Sequence
<220>
   <223> Description of Artificial Sequence: forward
   primer: MBU-O-567; mutagenesis ApaI site primer
<400> 24
   gctctaaaag ctgcgggccc agtaggaatt ctaatacg 38
<210> 25
   <211> 38
   <212> DNA
   <213> Artificial Sequence
<220>
   <223> Description of Artificial Sequence: reverse
   primer: MBU-O-568; mutagenesis ApaI site primer
<400> 25
   cgtattagaa ttcctactgg gcccgcagct tttagagc 38
<210> 26
   <211> 66
   <212> DNA
   <213> Artificial Sequence
<220>
   <223> Description of Artificial Sequence: forward
   primer: hgp130 primer
<400> 26
   gacgggcccg ccaccatgga ttacaaggat gacgacgata agatctcgac cgtggtacac 60
   agtggc 66
<210> 27
   <211> 29
   <212> DNA
   <213> Artificial Sequence
<220>
   <223> Description of Artificial Sequence: forward
   primer: MBU-O-675; hEpoR intr fragment primer
<400> 27
   ggcgagctcg gtgctggaca aatggttgc 29
<210> 28
   <211> 32
   <212> DNA
   <213> Artificial Sequence
<220>
   <223> Description of Artificial Sequence: reverse
   primer: MBU-O-676; hEpoR intr. fragment primer
<400> 28
   cgctctagat tactttaggt ggggtggggt ag 32
<210> 29
   <211> 29
   <212> DNA
   <213> Artificial Sequence
<220>
   <223> Description of Artificial Sequence: forward
   primer: MBU-O-677; mCIS primer
<400> 29
   gcggaattcg tcctctgcgt acagggatc 29
<210> 30
   <211> 30
   <212> DNA
   <213> Artificial Sequence
<220>
   <223> Description of Artificial Sequence: reverse
   primer: MBU-O-678; mCIS primer
<400> 30
   gcctctagat cagagttgga aggggtactg 30
<210> 31
   <211> 35
   <212> DNA
   <213> Artificial Sequence
<220>
   <223> Description of Artificial Sequence: forward
   primer: MBU-O-695; SV40LargeT primer
<400> 31
   gcgagatctc ggaagcagag gaaactaaac aactg 35
<210> 32
   <211> 32
   <212> DNA
   <213> Artificial Sequence
<220>
   <223> Description of Artificial Sequence: reverse
   primer: MBU-O-696; SV40LargeT primer
<400> 32
   gcgtctagat tatgtttcag gttcaggggg ag 32
<210> 33
   <211> 29
   <212> DNA
   <213> Artificial Sequence
<220>
   <223> Description of Artificial Sequence: forward
   primer: MBU-O-717; hEpoR Y426F mutagenesis
<400> 33
   ctgccagctt tgaattcact atcctggac 29
<210> 34
   <211> 29
   <212> DNA
   <213> Artificial Sequence
<220>
   <223> Description of Artificial Sequence: reverse
   primer: MBU-O-718; hEpoR Y426F mutagenesis
<400> 34
   gtccaggata gtgaattcaa agctggcag 29
<210> 35
   <211> 29
   <212> DNA
   <213> Artificial Sequence
<220>
   <223> Description of Artificial Sequence: forward
   primer: MBU-O-737; hVavS primer
<400> 35
   gcggaattca agctggagga atgttctca 29
<210> 36
   <211> 32
   <212> DNA
   <213> Artificial Sequence
<220>
   <223> Description of Artificial Sequence: reverse
   primer: MBU-O-738; hVavS primer
<400> 36
   cgcctcgagt tacacgtagt tggcagggaa cc 32
<210> 37
   <211> 32
   <212> DNA
   <213> Artificial Sequence
<220>
   <223> Description of Artificial Sequence: reverse
   primer: MBU-O-741; hVavS primer
<400> 37
   cgctctagat tacacgtagt tggcagggaa cc 32
<210> 38
   <211> 35
   <212> DNA
   <213> Artificial Sequence
<220>
   <223> Description of Artificial Sequence: forward
   primer: MBU-O-770; hGRB2 SH3 primer
<400> 38
   gcgagatctc gacatacgtc caggccctct ttgac 35
<210> 39
   <211> 31
   <212> DNA
   <213> Artificial Sequence
<220>
   <223> Description of Artificial Sequence: forward
   primer: MBU-O-771; hVavS primer
<400> 39
   gcgggatccc gaagctggag gaatgttctc a 31
<210> 40
   <211> 35
   <212> DNA
   <213> Artificial Sequence
<220>
   <223> Description of Artificial Sequence: forward
   primer: MBU-O-719; PuroR primer
<400> 40
   ccgctcgagc caccatggcc gagtacaagc ccacg 35
<210> 41
   <211> 27
   <212> DNA
   <213> Artificial Sequence
<220>
   <223> Description of Artificial Sequence: reverse
   primer: MBU-O-720; PuroR primer
<400> 41
   gctctagatt aggcaccggg cttgcgg 27
<210> 42
   <211> 27
   <212> DNA
   <213> Artificial Sequence
<220>
   <223> Description of Artificial Sequence: forward
   primer: MBU-O-766; gp130 primer
<400> 42
   gcgggatccg ccaccatgga ttacaag 27
<210> 43
   <211> 33
   <212> DNA
   <213> Artificial Sequence
<220>
   <223> Description of Artificial Sequence: reverse
   primer: MBU-O-769; hEpoR primer
<400> 43
   gcgcggccgc ttactttagg tggggtgggg tag 33
<210> 44
   <211> 24
   <212> DNA
   <213> Artificial Sequence
<220>
   <223> Description of Artificial Sequence: forward
   primer: MBU-O-835; ccdB primer
<400> 44
   cggaattcgc ttactaaaag ccag 24
<210> 45
   <211> 32
   <212> DNA
   <213> Artificial Sequence
<220>
   <223> Description of Artificial Sequence: reverse
   primer: MBU-O-836; ccdB primer
<400> 45
   atagtttagc ggccgctaat tctatattcc cc 32
<210> 46
   <211> 50
   <212> DNA
   <213> Artificial Sequence
<220>
   <223> Description of Artificial Sequence: forward
   primer: MBU-1094; Gateway-primer
<400> 46
   ccccaccact ttgtacaaga aagctgggtc tgcattcatt ttatgtttca 50
<210> 47
   <211> 48
   <212> DNA
   <213> Artificial Sequence
<220>
   <223> Description of Artificial Sequence: reverse
   primer: MBU-O-1076; Gateway-primer
<400> 47
   ggggacaagt ttgtacaaaa aagcaggcta cttaccacag actgtacg 48

## Description

The present invention relates to a recombinant receptor, comprising an extracellular ligand-binding domain and a cytoplasmic domain that comprises a heterologous bait polypeptide, which receptor is activated by binding of a ligand to said ligand binding domain and by binding of a prey polypeptide to said heterologous bait peptide. The present invention also relates to a method to detect compound-compound-binding using said recombinant receptor.

Protein-protein interactions are an essential key in all biological processes, from the replication and expression of genes to the morphogenesis of organisms. Protein-protein interactions govem amongst others ligand-receptor interaction and the subsequent signaling pathway; they are important in assembly of enzyme subunits, in the formation of biological supramolecular structures such as ribosomes, filaments and virus particles and in antigen-antibody interactions.

Researchers have developed several approaches in attempts to identify protein-protein interactions. Co-punfication of proteins and co-immunoprecipitation were amongst the first techniques used. However, these methods are tedious and do not allow high throughput screening. Moreover, they require lysis corrupting the normal cellular context. A major breakthrough was obtained by the introduction of the genetic approaches, of which the yeast two-hybrid (Fields and Song, 1989) is the most important one. Although this technique became widely used, it has several drawbacks.

The fusion proteins need to be translocated to the nucleus, which is not always evident. Proteins with intrinsic transcription activation properties may cause false positives. Moreover, interactions that are dependent upon secondary modifications of the protein such as phosphorylation cannot be easily detected.

Several altemative systems have been developed to solve one or more of these problems.

Approaches based on phage display do avoid the nuclear translocation. WO9002809 describes how a binding protein can be displayed on the surface of a genetic package, such as a filamentous phage, whereby the gene encoding the binding protein is packaged inside the phage. Phages, which bear the binding protein that recognizes the target molecule are isolated and amplified. Several improvements of the phage display approach have been proposed, as described e.g. in WO9220791, WO9710330 and W09732017.

However, all these methods suffer from the difficulties that are inherent at the phage display methodology: the proteins need to be exposed at the phage surface and are so exposed to an environment that is not physiological relevant for the *in vivo* interaction. Moreover, when screening a phage library, there will be a competition between the phages that results in a selection of the high affinity binders. Finally, modification-dependent phage display systems have not been described.

US5637463 describes an improvement of the yeast two-hybrid system, whereby can be screened for modification dependent protein-protein interactions. However, this method relies on the co-expression of the modifying enzyme, which will exert its activity in the cytoplasm and may modify other enzymes than the one involved in the protein-protein interaction, which may on its tum affect the viability of the host organism.

An interesting evolution is described in US5776689, by the so-called protein recruitment system. Protein-protein interactions are detected by recruitment of a guanine nucleotide exchange factor (Sos) to the plasma membrane, where Sos activates a Ras reporter molecule. This results in the survival of the cell that otherwise would not survive in the culture conditions used. Although this method has certainly the advantage that the protein-protein interaction takes place under physiological conditions in the submembranary space, it has several drawbacks. Modification-dependent interactions cannot be detected. Moreover, the method is using the pleiotropic Ras pathway, which may cause technical complications.

There is still a need for a selection system for protein-protein interactions that can study these interactions under physiological conditions, with a low and controllable background and by which modification-dependent protein-protein interactions can be isolated.

The present invention satisfies this need and provides additional advantages as well.

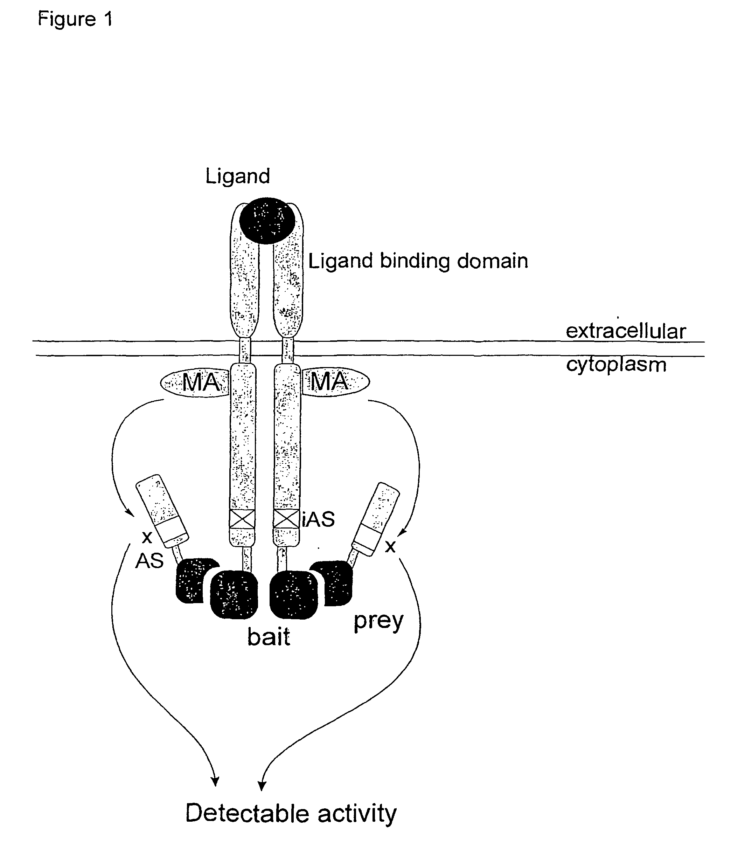

A schematic representation of the invention is given in Figure 1.

It is one aspect of the present invention to provide a recombinant transmembrane receptor, comprising an extracellular ligand binding domain and a cytoplasmic domain derived from a receptor, said cytoplasmic domain comprising a first interacting polypeptide, heterologous to said cytoplasmic domain, whereby said recombinant receptor is activated by binding of a ligand to said ligand binding domain and by binding of a second interacting polypeptide with the first interacting polypeptide. In this application, the first interacting polypeptide is called "bait" or "bait polypeptide", and the second interacting polypeptide is called "prey" or "prey polypeptide". The recombinant receptor can be a chimeric receptor, in which the ligand binding domain and the cytoplasmic domain are derived from two different receptors. Preferentially, the receptor is a multimerizing receptor, this can be a homomultimerizing receptor as well as a heteromultimerizing receptor. The cytoplasmic domain of the recombinant receptor comprises a heterologous bait polypeptide, which can be fused to the carboxyterminal end, or can replace a part of this carboxyterminal end or can be situated in the cytoplasmic domain itself, as an insertion or a replacement of an endogenous intemal fragment. In case of a heteromultimerizing receptor, not all the chains need to comprise the bait, but it is sufficient if one of the composing chains does comprise the bait in its cytoplasmic domain. At least one of the activation sites in the cytoplasmic domain of the receptor has been inactivated, so that the receptor is not activated and there is no active signaling pathway if only a ligand is binding to the ligand-binding domain of said recombinant receptor. Such inactivation can be obtained in several ways, such as by replacement of the amino acid, which can be activated, by another amino acid, by changing the amino acid context of the activation site or by deleting the activation site. Insertion of the heterologous bait polypeptide and inactivation of the activation sites may result in one or more deletions of the original cytoplasmic domain. The only limiting factor for the changes in the cytoplasmic domain is that said cytoplasmic domain should retain, directly or indirectly, its inherent modifying enzyme activity activity, either by retaining a modifying enzyme activity binding site such as a Jak binding site, or by incorporating an active modifying enzyme activity in the cytoplasmic domain itself. Activation of the receptor and of the signaling pathway is achieved by binding of a ligand to the ligand-binding domain and by binding of a prey polypeptide to the heterologous bait polypeptide comprised in the cytoplasmic domain of the receptor.

The gene, encoding the recombinant receptor comprising the bait polypeptide may be placed downstream either a constitutive or an inducible promoter. The latter construction may have some advantages in cases where there is a competition for the binding site between prey polypeptides and endogenous polypeptides. Induction of the recombinant receptor comprising the bait polypeptide in presence of the prey polypeptides may facilitate the binding and avoid saturation of the binding sites with endogenous polypeptides

One preferred embodiment is a recombinant receptor according to the invention whereby the activation site is a phosphorylation site and the modifying enzyme activity is a kinase.

Another preferred embodiment of the invention is a homomultimerizing recombinant leptin receptor, with a heterologous bait polypeptide fused into, or, preferentially, at the carboxyterminal end of its cytoplasmic domain. Said heterologous bait polypeptide may replace part of said cytopmasmic domain. Preferentially, the three conserved tyrosine phosphorylation sites of the cytoplasmic domain are inactivated, more preferentially by a replacement of tyrosine by phenylalanine. Another preferred embodiment is a homomultimerizing recombinant receptor in which an inactivated cytoplasmic domain of the leptin receptor, comprising a heterologous bait polypeptide, as described above, is fused to the ligand binding domain of the erythropoietin (EPO) receptor. Still another embodiment is a heteromultimerizing recombinant receptor in which the inactivated cytoplasmic domain of the leptin receptor, comprising a heterologous bait polypeptide is fused to the Interleukine-5 receptor α-chain ligand-binding domain for one subunit, and to the interleukine-5 receptor β-chain for another subunit. Still another embodiment is a heteromultimerizing recombinant receptor in which the inactivated cytoplasmic domain of the leptin receptor, comprising a heterologous bait polypeptide is fused to the GM-CSFα-chain ligand-binding domain for one subunit, and to the interleukine-5 receptor β-chain for another subunit.

It is another aspect of the invention to provide a recombinant receptor, comprising a ligand binding domain and a cytoplasmic domain that comprises a heterologous bait polypeptide which can be modified by modifications such as, but not limited to phosphorylation, acetylation, acylation, methylation, ubiquitinilation, glycosylation or proteolytic processing, whereby said recombinant receptor is activated by binding of a ligand to said ligand binding domain and by binding of a prey polypeptide to said heterologous bait polypeptide and whereby said binding of the prey polypeptide to the heterologous bait polypeptide is dependent upon the modification state of the heterologous bait polypeptide, i.e. either there is only binding with modification, or there is only binding without modification. Said modification state can be, but is not limited to presence or absence of phosphorylation, acetylation, acylation, methylation, ubiquitinilation or glycosylation, or occurrence of proteolytic cleavage or not. The bait is modified by the bait-modifying-enzyme activity which can be, but is not necessarily identical to the modifying enzyme activity which is modifying the activation site. The recombinant receptor can be a chimeric receptor, in which the ligand binding domain and the cytoplasmic domain are derived from two different receptors. Preferentially, the receptor is a multimerizing receptor. As described above, the cytoplasmic domain of the recombinant receptor comprises a heterologous bait polypeptide, which can be fused to the carboxyterminal end, or can replace a part of this carboxyterminal end or can be situated in the cytoplasmic domain itself, as an insertion or a replacement of an endogenous intemal fragment. In case of a heteromultimerizing receptor, not all the chains need to comprise the bait, but it is sufficient if one of the composing chains does comprise the bait in its cytoplasmic domain. At least one of the activation sites in the cytoplasmic domain of the receptor has been inactivated, so that the receptor is not activated and there is no active signaling pathway if only a ligand is binding to the ligand-binding domain of said recombinant receptor. Such inactivation can be obtained in several ways, such as by replacement of the amino acid, which can be activated, by another amino acid, or by changing the amino acid context of the activation site or by deleting the activation site. Insertion of the heterologous bait polypeptide and inactivation of the activation sites may result in one or more deletions of the original cytoplasmic domain. The only limiting factor for the changes in the cytoplasmic domain is that said cytoplasmic domain should retain, directly or indirectly, its inherent modifying enzyme activity, either by retaining a modifying enzyme binding site, or by incorporating an active modifying enzyme activity in the cytoplasmic domain itself. Preferentially, the activation site is a phosphorylation site, and the modifying enzyme activity is a kinase activity.

The modification of the bait may be either in cis or in trans, i.e. by an enzymatic activity that is situated on the same cytoplasmic domain, or by an enzymatic activity that comes from elsewhere. Preferentially, the modification of the bait is induced by binding of a ligand to the ligand-binding domain. One preferred embodiment is a homodimerizing receptor in which the bait is phosphorylated by the inherent kinase activity of the cytoplasmic domain, preferentially a Jak kinase that is binding to said cytoplasmic domain. Another preferred embodiment is a heteromultimerizing receptor where the cytoplasmic domain of one chain comprises a bait to be modified, and the cytoplasmic domain of another chain comprises the bait-modifying enzyme activity.

Activation of the receptor and of the signaling pathway is achieved by binding of a ligand to the ligand-binding domain and by binding of a prey polypeptide to the heterologous bait polypeptide situated in the cytoplasmic domain of the receptor.

Binding of said prey polypeptide is dependent upon the modification state of said heterologous bait polypeptide, it means that binding occurs only in case the bait is modified or only in case the bait is not modified.

It is another aspect of the invention to provide a prey polypeptide; whereby said prey polypeptide is a fusion protein comprising a polypeptide that can interact directly or indirectly with a bait polypeptide and another polypeptide that comprises at least one activation site of a receptor. Said activation site is preferentially a phosphorylation site, more preferentially a tyrosine phosphorylation site. Even more preferentially, said tyrosine phosphorylation site is part of a Signal Transducer and Activator of Transcription (STAT) binding site, most preferentially part of a STAT1 and/or STAT3 binding site. Direct interaction means that there is a direct protein-protein contact between the heterologous bait polypeptide and the prey polypeptide; indirect interaction means that the heterologous bait polypeptide interacts with one or more other polypeptides to form a complex that interacts with said prey polypeptide or vice versa. In the latter case, the prey polypeptide may interact either with only one or with several polypeptides from the complex. The binding of the prey polypeptide to the bait polypeptide may be dependent upon the modification state of said bait polypeptide and/or of proteins within the binding complex.

In case that interactions of nuclear proteins are studied, the prey polypeptide may comprise a Nuclear Export Sequence (NES), to ensure that it is available in the cytosol. The NES signal (amino acids 37-46) of the heat-stable inhibitor of the cAMP-dependent protein kinase has been shown to override a strong nuclear localisation signal (Wiley *et al*., 1999). This NES will keep the prey polypeptide in the cytoplasm even if it has a strong nuclear localisation signal, facilitating the interaction with the bait.

One preferred embodiment is a prey polypeptide according to the invention, whereby said prey polypeptide interacts with the heterologous bait polypeptide of a recombinant receptor according to the invention. Upon binding of a ligand to the ligand binding domain of the recombinant receptor and upon direct or indirect interaction of said heterologous bait polypeptide with said prey polypeptide, the activation site of the prey polypeptide can be modified by the modifying enzyme activity inherent to the cytoplasmic domain of the receptor. The modification of the activation site will activate the signaling pathway. Preferentially, said activation site is a phosphorylation site and the modifying enzyme activity is a kinase activity. More preferentially, this activation comprises binding of a STAT polypeptide to the phosphorylated phosphorylation site, followed by phosphorylation of said STAT polypeptide and subsequent dimerization of two phosphorylated STAT molecules.

Another aspect of the invention is a vector, encoding a recombinant receptor according to the invention and/or a vector, encoding a prey polypeptide according to the invention. Said recombinant receptor and said prey polypeptide may be situated on one or on separated vectors. The vector can be any vector, know to the person skilled in the art, including but not limited to episomal vectors, integrative vectors and viral vectors. A preferred embodiment is a bait vector whereby the bait may be integrated in the chromosome by a recombinase-assisted integration such as cre-lox or flp-frt, and/or a retroviral prey vector that allows retroviral integration in the genome.

Another aspect of the invention is an eukaryotic cell comprising a recombinant receptor according to the invention. Preferentially, the eukaryotic cell is obtained by transformation or transfection with one or more vectors according to the invention. Said eukaryotic cell comprises, but is not limited to yeast cells, fungal cells, plant cells, insect cells and mammalian cells. Preferentially, the eukaryotic cell is a mammalian cell. A preferred embodiment is an eukaryotic cell line expression the mouse retroviral receptor, allowing safe retroviral work using retroviral cDNA libraries.

Still another aspect of the invention is a kit, comprising one or more cloning vectors allowing the construction of one or more vectors according to the invention. It is clear for the people skilled in the art that a cloning vector, encoding a recombinant receptor in which the part, encoding for the cytoplasmic domain comprises one or more restriction sites allowing an "in frame" fusion of a nucleic acid fragment encoding a polypeptide can easily be used to construct a vector encoding a recombinant receptor according to the invention. In a similar way, a cloning vector encoding a first polypeptide comprising at least one activation site, comprising one or more restriction sites allowing an "in frame" fusion of a nucleic acid encoding a second polypeptide with said first polypeptide can easily be used to construct a vector encoding a prey polypeptide according to the invention. Altematively, both for the construction of the vector encoding the recombinant receptor and for the vector encoding the prey polypeptide, other cloning strategies known to the person skilled in the art may be used.

Still another aspect of the invention is a method to detect compound-compound binding using a recombinant receptor and/or a prey polypeptide according to the invention. In a preferred embodiment, an eukaryotic cell, carrying a recombinant receptor according to the invention is transformed or transfected with a vector library encoding prey polypeptides according to the invention. Bait-prey binding will result in an activation of the signaling pathway and can be detected by the use of a reporter system. Although it is not an essential feature, the use of a chimeric receptor may represent an additional advantage for this method. A first advantage of the use of a chimeric receptor in this method is that it allows the elimination of a non bait-specific background. Indeed, by the use of two different receptors, a non bait-comprising and a bait-comprising receptor, a difference can be made between bait-specific and non bait-specific binding. This can be realized by the use of a host cell carrying at least two receptors, a first receptor, comprising a first ligand binding domain and a cytoplasmic domain that does not comprise an activation site neither a heterologous bait polypeptide and a second receptor, comprising the same inactivated cytoplasmic domain, however with a heterologous bait polypeptide now, and a second ligand binding domain. Upon exogenous addition of the first ligand to the medium and binding of the first ligand to the receptor, a positive signal can only be detected when there is a non bait-specific interaction of a prey polypeptide fused to a polypeptide comprising an activation site with the cytoplasmic domain of the receptor; these cells can be selected and/or eliminated. After selection and/or elimination of the non bait-specific interacting preys, the second ligand can be added to the medium. Upon binding of the second ligand to its ligand-binding domain, a positive signal will only be detected upon specific bait-prey interaction, as the preys binding to the cytoplasmic domain have been removed. Another advantage of the use of a chimeric receptor is that, in a similar way, a subtractive selection can be made for preys binding to closely related but different baits.

One specific embodiment of the method to detect compound-compound binding is a method whereby said binding is a protein-protein interaction. Another specific embodiment is a method to detect protein-protein interaction, whereby said interaction is modification state dependent. Still another specific embodiment is a method to detect compound-compound binding, whereby said binding is mediated by three or more partners. In this case, one or more partners may not be or not completely be of proteineous nature. It is clear for a person skilled in the art that a recombinant receptor, according to the invention may, as a non-limiting example, bind to a small molecule. On the other hand, the prey polypeptide, according to the invention may also bind to the small molecule, so that bait and prey are linked together by said small molecule. Said small molecule may be present in the host cell, as a compound produced by the cell itself, or as a compound that is taken up from the medium.

Preferably, said method to detect compound-compound binding comprises the construction of an eukaryotic cell comprising a recombinant receptor according to the invention, followed by transformation or transfection of said cell by a library of prey polypeptide vectors according to the invention. The compound-compound binding is detected by the activation of the receptor, leading to an active signaling pathway, resulting in the induction of a reporter system. A reporter system can be any system that allows the detection and/or the selection of the cells carrying a recombinant receptor according to the invention. It is clear for the person skilled in the art that several reporter systems can be used. As a non-limiting example, a luciferase gene, an antibiotic resistance gene or a cell surface marker gene can be placed after a promoter that is induced by the signaling pathway. Alternatively, reporter systems may be used that are based on the change in characteristics of compounds of the signaling pathway, when said pathway is active, such as the phosphorylation and/or dimerisation of such compounds.

### Definitions

The following definitions are set forth to illustrate and define the meaning and scope of various terms used to describe the invention herein.

*Receptor* as used here does not necessarily indicate a single polypeptide, but may indicate a receptor complex, consisting of two or more polypeptides, and comprising a ligand binding domain and a cytoplasmic domain. Recombinant receptor means that at least one of said polypeptides is recombinant. Preferably the polypeptide comprising the cytoplasmic domain is recombinant.

*Activation site* of a receptor is the site that, in the wild type receptor, is modified after binding of a ligand to the ligand binding domain, leading to a reorganization of the receptor and subsequent activation of the modifying enzyme activity, and to which a compound of the signaling pathway can bind after modification, or any site that can fulfill a similar function.

In the latter case, the activation site is not necessarily located on the same polypeptide as in the wild type receptor, but may be situated on another polypeptide of the receptor complex.

*Modifying enzyme activity* as used here means the enzymatic activity, associated to or incorporated in the cytoplasmic domain of the receptor that is normally induced upon binding of the ligand to the ligand binding domain and subsequent reorganization of the receptor (e.g. by a conformational change), and may modify the activation site. Preferably, the activation site is a phosphorylation site and the modifying enzyme activity is a kinase activity. The bait-modifying enzyme activity means the activity which modifies the bait. It can be, but is not necessarily identical to the modifying enzyme activity.

*Activation of a receptor* as used here means that the receptor is inducing a signaling pathway, by binding of a compound of the signaling pathway to the modified activation site, whereby said activation normally results in the induction or repression of one or more genes. Said gene is preferentially a reporter gene, which allows monitoring the activation of the receptor. An *activated receptor* is a receptor where the binding of a compound to the activation site has been enabled by modification of said site. A receptor in which the modifying enzyme activity has been induced, without modification of an activation site is not considered as activated.

*Multimerizing receptor* as used here means that the activated receptor comprises several polypeptides. It does not necessarily imply that the multimerization is induced by ligand binding: the receptor can exist as a preformed complex of which the conformation is changed upon ligand binding.

*Polypeptide* as used here means any proteineous structure, independent of the length and includes molecules such as peptides, phosphorylated proteins and glycosylated proteins. Polypeptide as used herein is not necessarily indicating an independent compound but can also be used to indicate a part of a bigger compound, such as a domain of a protein.

*Heterologous bait polypeptide,* as *comprised in the cytoplasmic domain of a receptor means that within the cytoplasmic domain, or fused to the cytoplasmic domain, there is a polypeptide that is not present in the cytoplasmic domain of the non-recombinant receptor. Said heterologous bait polypeptide may replace a part of said cytoplasmic domain. Bait herein means that this polypeptide can interact with other polypeptides, not belonging to the normal receptor complex.*

*Prey polypeptide* as used here means a fusion protein comprising a polypeptide that can bind with the heterologous bait polypeptide and a polypeptide that comprises at least one activation site.

*Ligand* means every compound that can bind to the extracellular domain of a receptor and that is able to initiate the signaling pathway by binding to said extracellular domain. Initiating as used here means starting the events that normally directly follow the binding of the ligand to the extracellular domain of a receptor, e.g. multimerization for a multimerizing receptor, but it does not imply activation of the receptor and/or accomplishing of the signaling pathway.

*Compound* means any chemical or biological compound, including simple or complex organic or inorganic molecules, peptides, peptido-mimetics, proteins, antibodies, carbohydrates, nucleic acids or derivatives thereof.

*Bind(ing)* means any interaction, be it direct or indirect. A direct interaction implies a contact between the binding partners. An indirect interaction means any interaction whereby the interaction partners interact in a complex of more than two compounds. This interaction can be completely indirect, with the help of one or more bridging compounds, or partly indirect, where there is still a direct contact that is stabilized by the interaction of one or more compounds.

*Functional fragment of the inactivated leptin receptor cytoplasmic domain* means a fragment of the leptin receptor cytoplasmic domain that still allows binding of the Jak kinases.

*Inactivation of an activation site* means any change, mutation or deletion that is inhibiting a modification at the position of the potentially modified residue in the polypeptide. In particular, inactivation of a tyrosine phosphorylation site means any change, mutation or deletion that is inhibiting a phosphorylation at the position of the potentially phosphorylated tyrosine residue in the polypeptide. Preferentially, it is a mutation at this position; more preferentially, it is a change of tyrosine into phenylalanine.

*Cloning vector* is a vector that is generally considered as an intermediate step for the construction of another vector. It is intended to insert one or more nucleic acid fragments, in order to obtain one or more new vectors that will be used to transform or transfect the host cell of interest, or as cloning vectors themselves.

### Brief description of the figures

**Figure 1:** Principle of the receptor-based interaction trap. Ligand binding leads to activation of a modifying enzyme activity (MA). Due to the inactivation of the normal receptor activation site (inactivated activation site, iAS), the activation of modifying enzyme activity does not result in an activation of the signaling pathway, unless the heterologous bait in the cytoplasmic domain of the recombinant receptor (indicated as 'bait') is binding to a prey polypeptide (indicated as 'prey') which is fused to a polypeptide comprising an activation site (AS). The modifying enzyme activity can now modify this activation site; modification (x) of this activation site results in activation of the signaling pathway and induction of a reporter system (indicated as 'detectable activity').
**Figure 2**: Functionality of EpoR-LepR chimera in the Hek293T PAP21 cell line, as measured by luciferase light emission, measured in a chemiluminescence counter (counts per second, cps). The cells were transfected with:
   a. pSV-SPORT + pMET7mcs + pGL3-rPAP1-luci + pUT651
   b. pSV-SPORT EpoR/LepR + pMET7mcs + pGL3-rPAP1-luci + pUT651
   c. pMET7 LepRY985/1077F + pMET7mcs + pGL3-rPAP1-luci +pUT651
   NC: non-stimulated negative control. Stimulations were carried out as described in the examples.
**Figure 3**: Functionality of p53-SV40 LargeT interaction trap, as measured by luciferase light emission, measured in a chemiluminescence counter (cps).
   The cells were transfected with:
   a. pSV-SPORT + pMG1-SVT + pGL3-rPAP1-luci + pUT651
   b. pSV-SPORT + pMG1-CIS + pGL3-rPAPl-luci + pUT651
   c. pSV-SPORT + pMET7-SVT + pGL3-rPAP1-luci + pUT651
   d. pSEL1-p53 + pMG1-SVT + pGL3-rPAP1-luci + pUT651
   e. pSEL1-p53 + pMG1-CIS + pGL3-rPAP1-luci + pUT651
   f. pSEL1-p53 + pMET7-SVT + pGL3-rPAP1-luci + pUT651
   NC: non-stimulated negative control. Stimulations were carried out as described in the examples.
**Figure 4:** Functionality of the EpoR-CIS phosphorylation-dependent interaction trap, as measured by luciferase light emission, measured in a chemiluminescence counter (cps).
   The cells were transfected with:
   a. pSV-SPORT + pMG1-CIS + pGL3-rPAP1-luci + pUT651
   b. pSV-SPORT + pMG1-SVT + pGL3-rPAP1-luci + pUT651
   c. pSV-SPORT + pEF-FLAG-I/mCIS + pGL3-rPAP1-luci + pUT651
   d. pSEL1-EpoR + pMG1-CIS + pGL3-rPAP1-luci + pUT651
   e. pSEL1-EpoR + pEF-FLAG-I/mCIS + pGL3-rPAP1-luci + pUT651
   f. pSEL1-EpoRY-F + pMG1-CIS + pGL3-rPAP1-luci + pUT651
   g. pSEL1-EpoR + pMG1-SVT + pGL3-rPAP1-luci + pUT651
   NC: non-stimulated negative control. Stimulations were carried out as described in the examples.

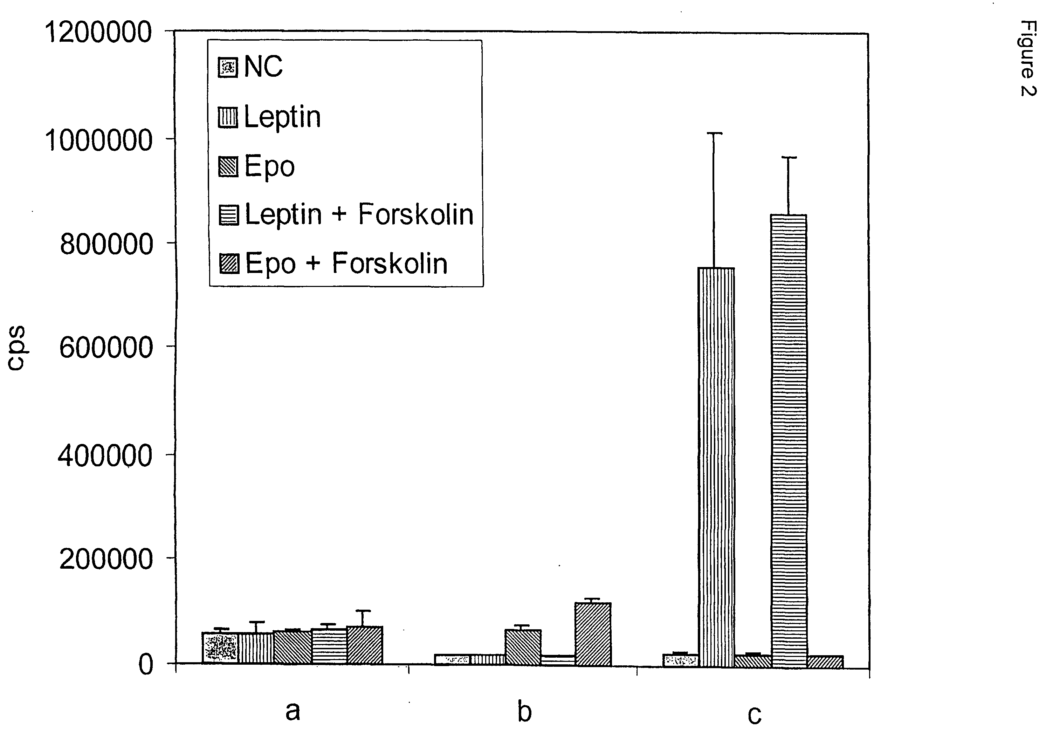

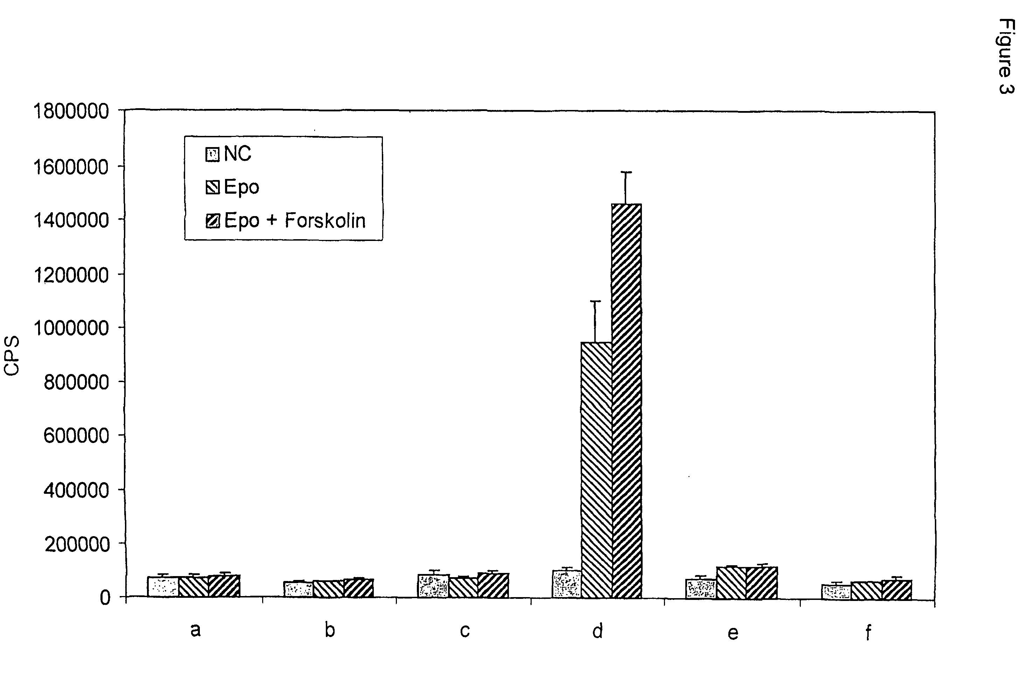

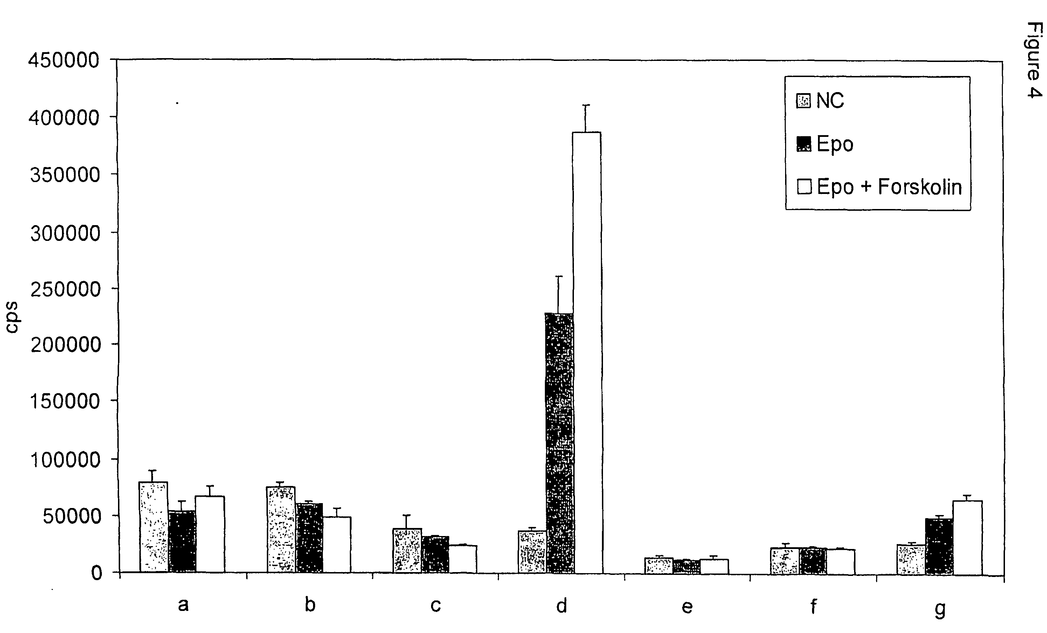

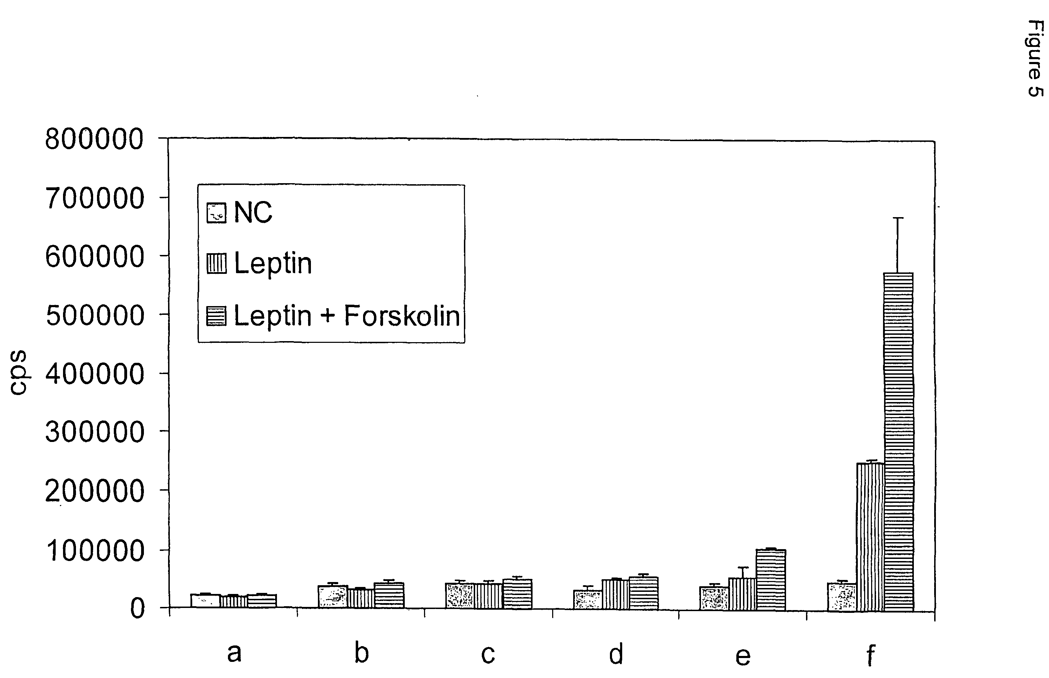

**Figure 5:** Functionality of the IRS1-GRB2-Vav indirect interaction trap, as measured by luciferase light emission, measured in a chemiluminescence counter (cps).
   The cells were transfected with:
   a. pMET7mcs + pMG1-CIS + pGL3-rPAP1-luci + pUT651
   b. pMET7mcs + pMG1-GRB2S + pGL3-rPAP1-luci + pUT651
   c. pMET7mcs + pMG1-VavS + pGL3-rPAPl-luci + pUT651
   d. pMET7 LepR-IRS1 + pMG1-CIS + pGL3-rPAP1-luci + pUT651
   e. pMET7 LepR-IRS1 + pMG1-GRB2S + pGL3-rPAP1-luci + pUT651
   f. pMET7 LepR-IRS1 + pMG1-VavS + pGL3-rPAP1-luci + pUT651
   NC: non-stimulated negative control. Stimulations were carried out as described in the examples.

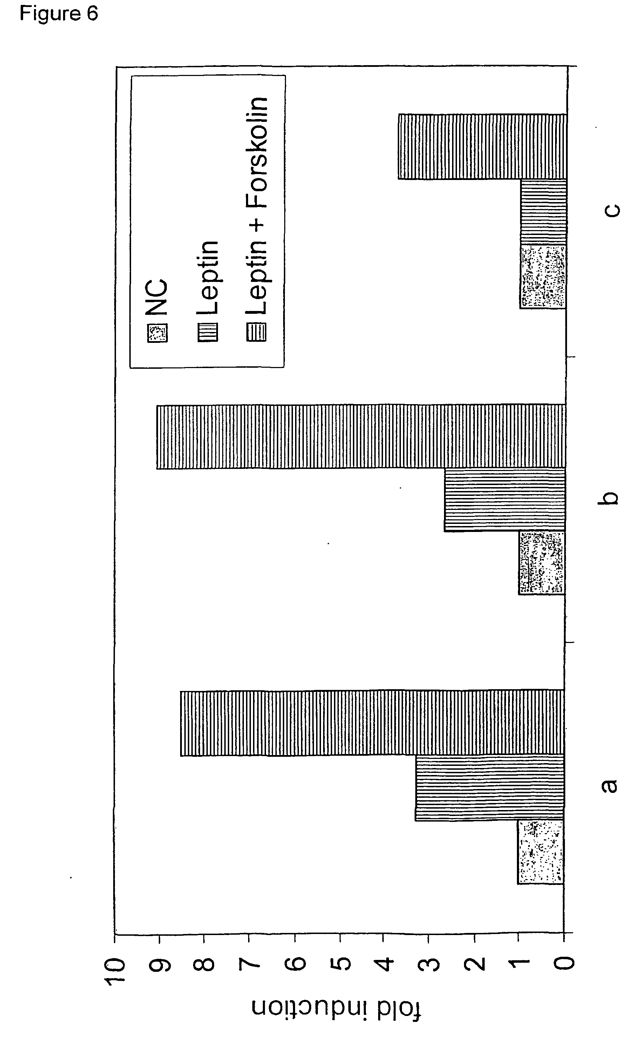

**Figure 6:** Functionality of the IRS1-GRB2-Vav indirect interaction trap, as measured by luciferase light emission, measured in a chemiluminescence counter (cps): GRB2 dose dependent inhibition of the signal.
   The cells were transfected with:
   a. pMET7 LepR-IRS1 + 200 ng pMG1-VavS + pGL3-rPAP1-luci + pUT651
   b. pMET7 LepR-IRS1 + 200 ng pMG1-VavS + 200 ng pMET7 GRB2SH3 + pGL3-rPAP1-luci + pUT651
   c. pMET7 LepR-IRS1 + 200 ng pMG1-VavS + 1000 ng pMET7 GRB2SH3 + pGL3-rPAP1-luci + pUT651
   NC: non-stimulated negative control. Stimulations were carried out as described in the examples.

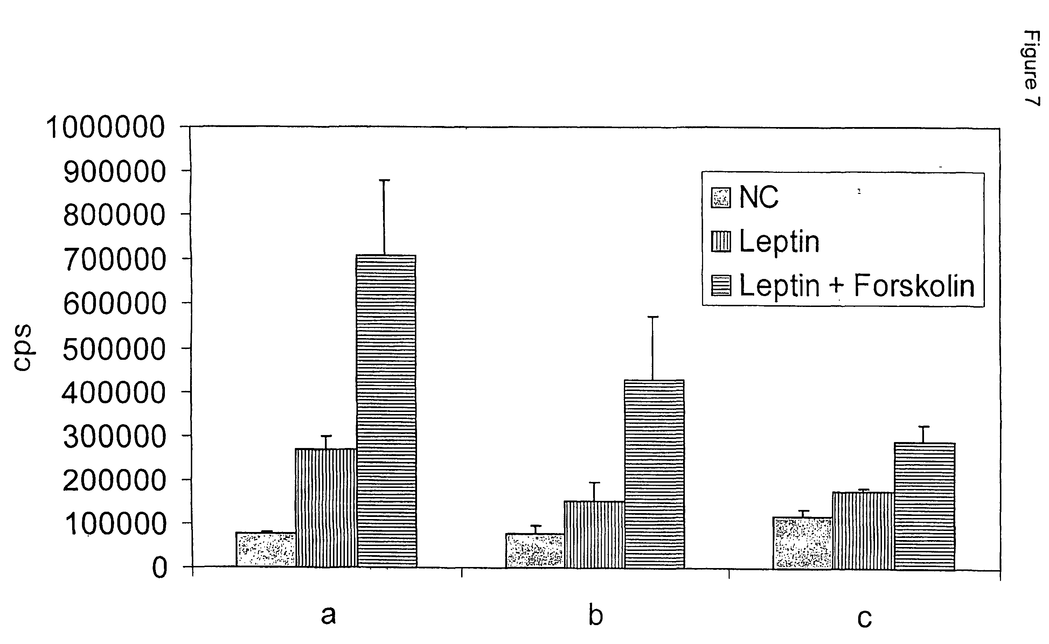

**Figure 7**: Functionality of the IRS1-GRB2-Vav indirect interaction trap, as measured by luciferase light emission, measured in a chemiluminescence counter (cps): VavS dose dependent inhibition of the signal.
   The cells were transfected with:
   a. pMET7 LepR-IRS1 + 200 ng pMG1-VavS + pGL3-rPAP1-luci + pUT651
   b. pMET7 LepR-IRS1 + 200 ng pMG1-VavS + 200 ng pMET7 VavS + pGL3-rPAP1-luci + pUT651
   c. pMET7 LepR-IRS1 + 200 ng pMG1-VavS + 1000 ng pMET7 VavS + pGL3-rPAP1-luci + pUT651
   NC: non-stimulated negative control. Stimulations were carried out as described in the examples.

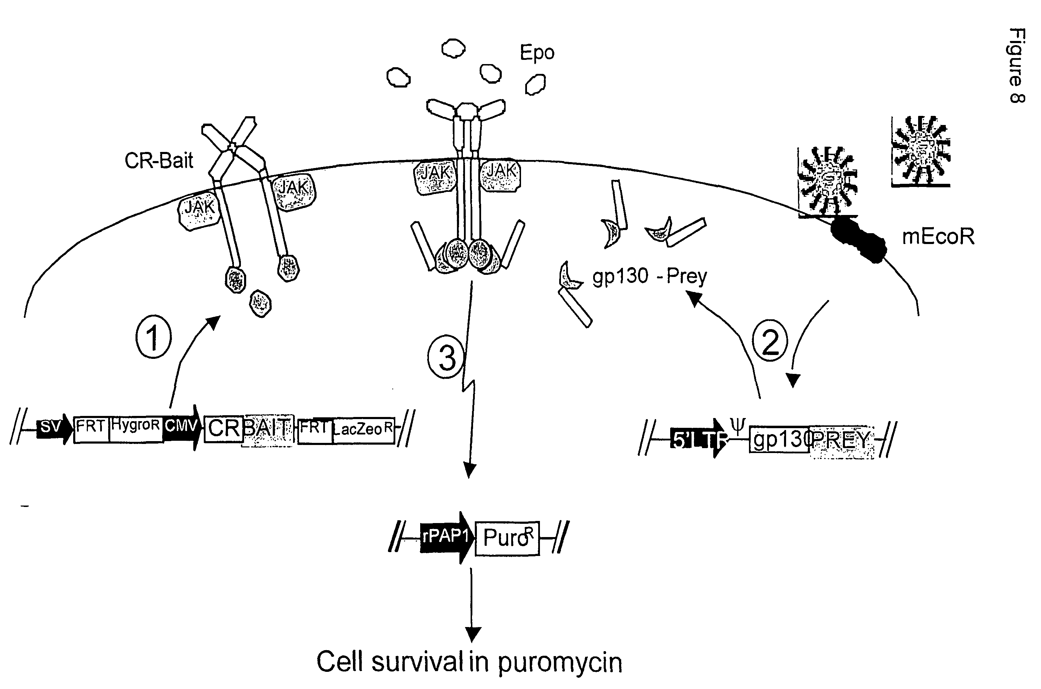

**Figure 8**: Layout of an optimised MAPPIT-based two-hybrid screening method
   The procedure encompasses three successive steps, indicated with encircled numbers. First, cells expressing the chimeric receptor with the C-terminal "bait" (CR-Bait) are generated upon recombinase-assisted genomic integration, followed by hygromycin selection. Next, gp130-"prey" chimeras are expressed upon retroviral gene transfer. Finally, if cognate "bait"-"prey" interaction occurs, ligand binding induces a signalling cascade leading to induction of the puromycin resistance marker and concomitant formation of cell colonies in selective medium. Direct RT-PCR amplification of "prey" encoding transcripts from lysed cell colonies allows rapid "prey" identification.

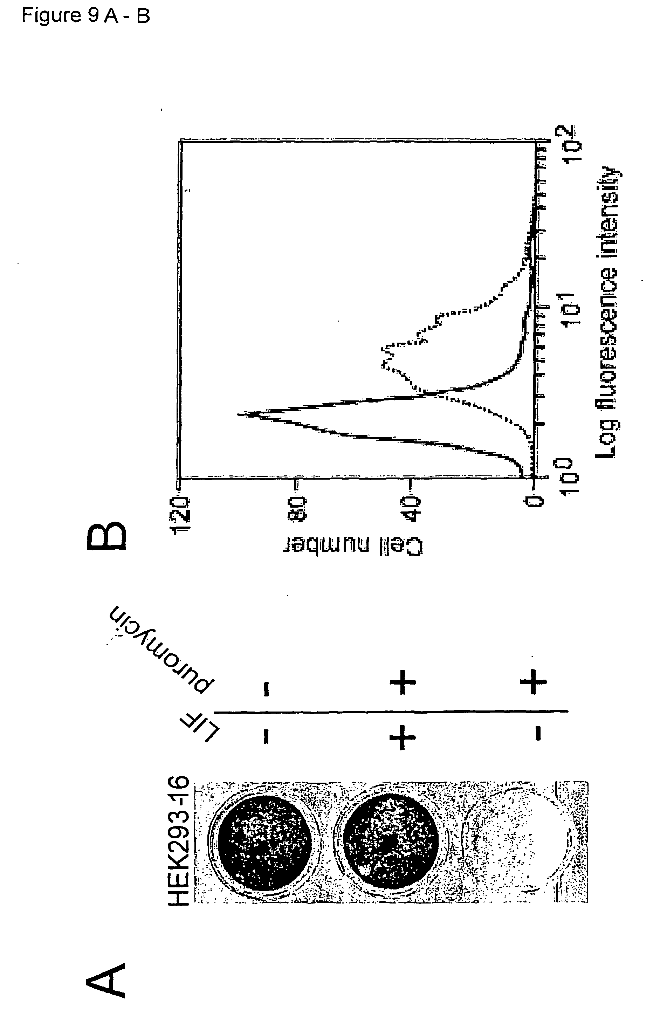

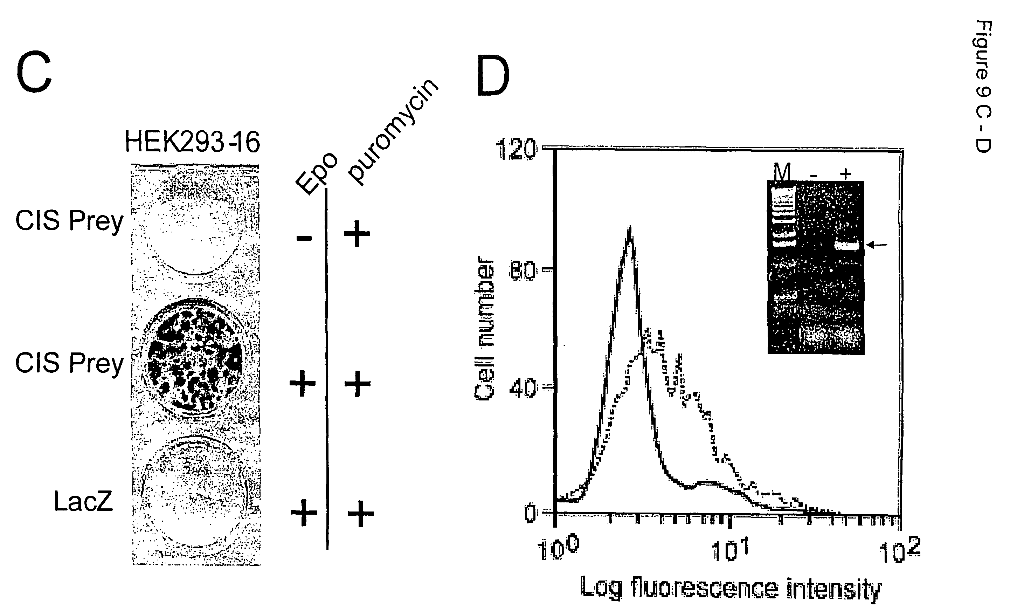

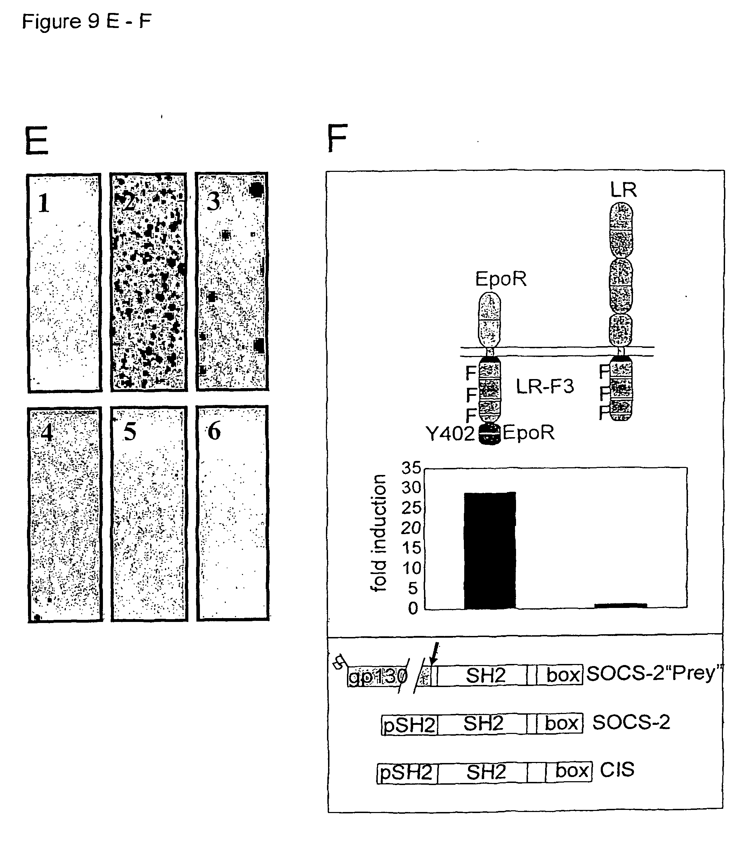

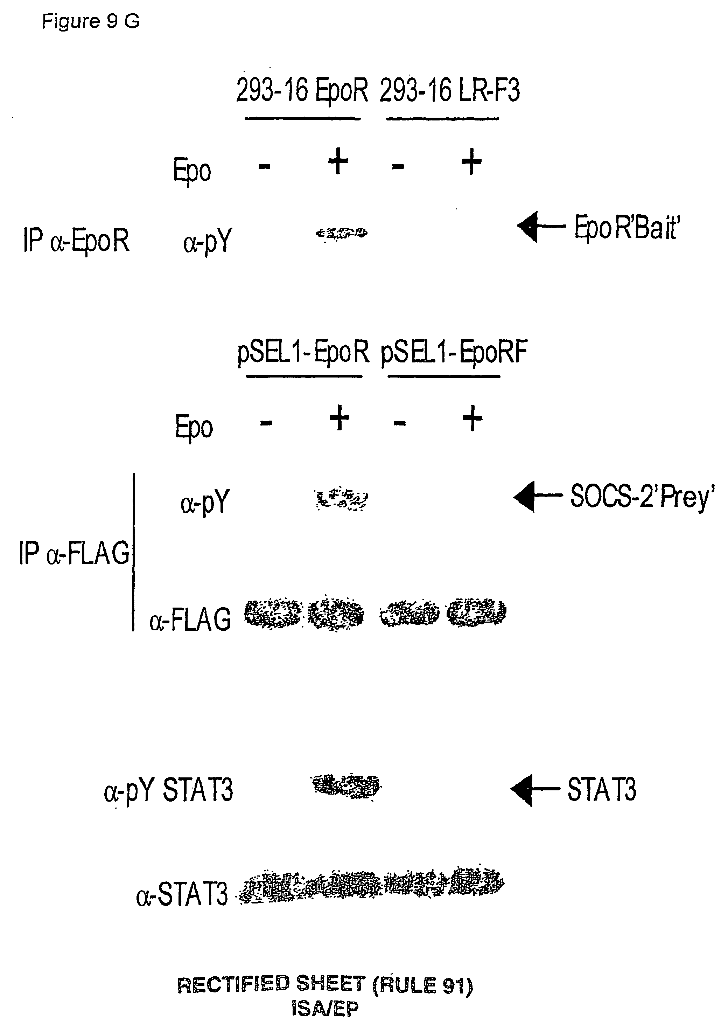

**Figure 9**: the MAPPIT procedure for two-hybrid screening
   (A) *The HEK293-16 cell line shows ligand-induced puromycin resistance.* HEK293-16 cells were seeded in a 24 well plate, and were left untreated (top well), or were subject to puromycin selection (1 µg/ml) with (middle well) or without (bottom well) prior activation of gp130 using LIF for 48 hours. After 1 week, surviving cells were stained with crystal violet.
   *(B) Selection of isogenic HEK293-16 cells expressing the EpoR"bait"*. HEK293-16 cells were co-transfected with the pcDNA5/FRT-EpoR"bait" and the Flp recombinase expression vectors and selected for hygromycin resistance (100 µg/ml) for 10 days. Cells were stained using polyclonal antiserum recognising the extracellular domain of the EpoR and Alexa488-labelled secondary antibody. Solid and dotted lines show parental or hygromycin-selected HEK293-16 cells, respectively.
   *(C) Selection of cells based on a cognate "prey"-"bait" interaction.* Hygromycin-resistant cells from (B) were seeded in a 24 well plate, infected with CIS"prey" expressing retrovirus (1/30 dilution of retroviral stock) for 48 hours, and were either left untreated (top well) or were stimulated with Epo for another 48hours (middle well), prior to treatment with puromycin (1 µg/ml). The bottom well shows Epo-stimulated cells selected as above, but expressing irrelevant lacZ protein (1/3 dilution of retroviral stock). Surviving cells were stained after 7 days with crystal violet.
   *(D) Puromycin-resistant cells express the "prey" chimera.* Parental HEK293-16 cells or puromycin-resistant cells from (C) were permeabilized, sequentially treated with anti-FLAG antibody and FITC-labelled secondary antibody and subjected to FACS analysis. Solid and dotted lines show parental or puromycin-selected HEK293-16 cells, respectively. (INSET) *RT-PCR detection of transcripts encoding the "prey" chimera.* Cells from (C) were lysed and the "prey"-encoding transcript was amplified by RT-PCR. The arrow indicates the CIS-specific amplicon, which was verified by DNA sequencing. A negative control on parental cells was also performed (middle lane). M: marker lane.
   *(E) Dose-dependent recovery of gp130-CIS"prey" expressing cell clones*. EpoR"bait" expressing cells were seeded in 75 cm² culture flasks and infected with CIS"prey" expressing retrovirus, 1/10 serially diluted in a complex retroviral HEK293 cDNA library. After selection, puromycin-resistant colonies were stained using crystal violet. Panel 1 shows cells infected with a 1/10 dilution of CIS-"prey" but without Epo stimulation. Panels 2-5 show the 1/10 to 1/10,000 serial dilutions, while panel 6 shows the outcome of cells infected with the retroviral cDNA library in the absence of CIS"prey". In a parallel "spiking" experiment, 19 out of 21 analysed clones contained gp130-CIS"prey" transcripts.
   *(F) Functional analysis of the EpoR"bait" - SOCS-2"prey" interaction.* MAPPIT screening of a HEK293 cDNA library using the EpoR"bait" resulted in the isolation of a cell clone expressing a SOCS-2"prey" construct. This selected clone was transiently transfected with the pXP2d2-rPAP1-luci reporter alone, or in combination with the vector encoding the LR-F3 variant, and was stimulated with Epo or leptin for 24 hours, respectively, or left unstimulated. The upper panel shows the corresponding luciferase inductions with a diagrammatic presentation of the activated receptors on top. In the lower panel, a graphic representation of the SOCS-2"preyr" chimera and of intact SOCS-2 and CIS is shown.
   *(G) The EpoR"bait"- SOCS-2"prey" interaction is phosphorylation dependent.* (Upper panel) HEK293-16 cells expressing chimeric receptors with (293-16 EpoR) or without (293-16 LR-F3) the EpoR"bait" were stimulated with Epo or were left untreated. Ligand-dependent phosphorylation of the EpoR"bait" is observed, in contrast to lack of phosphorylation of the LR-F3 chimera.
      (Middle and lower panels) HEK293T cells were transiently transfected with expression constructs for the EpoR"bait" (pSEL1-EpoR) and for the SOCS-2"prey". The middle panel and lower panel respectively show ligand-dependent "prey" and STAT3 phosphorylation which is only observed upon transfection with pSEL1-EpoR but not with pSEL1-EpoRF. Expression controls for the SOCS-2"prey" and for STAT3 are also shown.

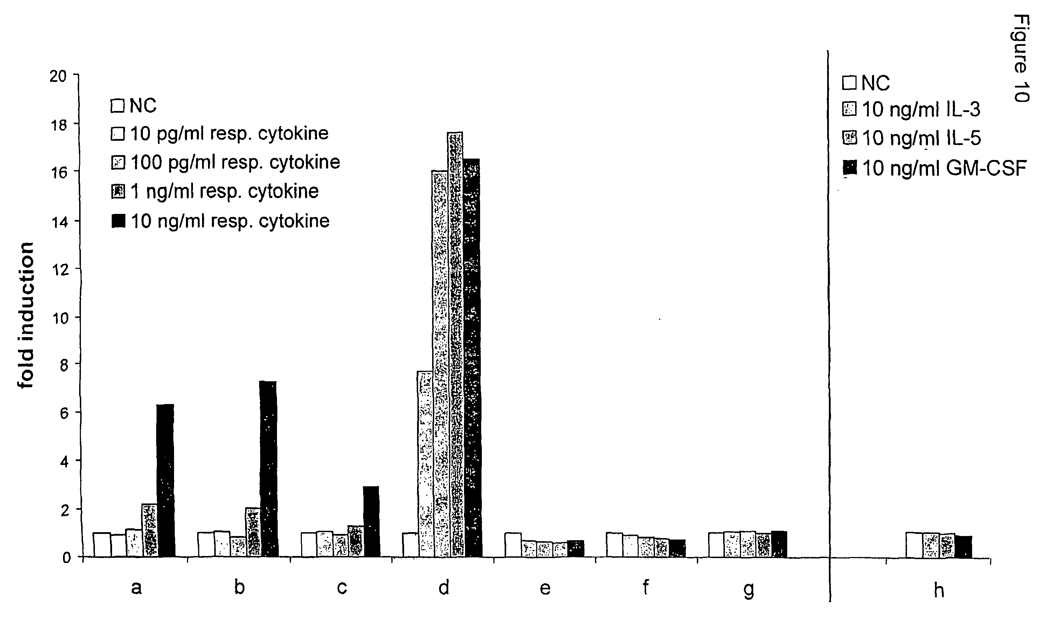

**Figure 10:** Functionality of IL3R-, IL5R- and GM-CSFR-LepR chimera in the Hek 293T cell line, as as measured by luciferase light emission, measured in a chemiluminescence counter (cps).
   The cells were transfected with:
   a. pSV-SPORT-EpoR/LepR + pGL3-rPAP1-luci + pUT651
   b. pSV-SPORT-IL-3Rα/LepR + pSV-SPORT-β_{c}/LepR + pGL3-rPAP1-tuci + pUT651
   c. pSV-SPORT-IL-5Rα/LepR + pSV-SPORT-β_{c}/LepR + pGL3-rPAP1-luci + pUT651
   d. pSV-SPORT-GM-CSFRα/LepR + pSV-SPORT-β_{c}/LepR + pGL3-rPAP1-luci + pUT651
   e. pSV-SPORT-IL-3Rα/LepR + pGL3-rPAP1-luci + pUT651
   f. pSV-SPORT-IL-5Rα/LepR + pGL3-rPAP1-luci + pUT651
   g. pSV-SPORT-GM-CSFRα/LepR + pGL3-rPAP1-luci + pUT651
   h. pSV-SPORT-β_{c}/LepR + pGL3-rPAP1-luci + pUT651
   NC: non-stimulated negative control. Stimulation was as described in the examples.

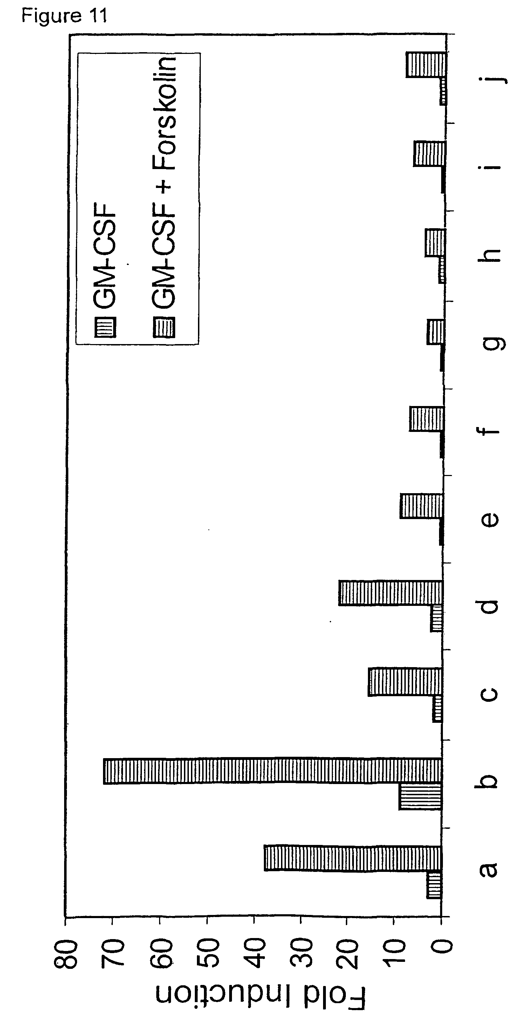

**Figure 11**: Functionality of the Smad3-Smad4 phosphorylation-dependent interaction trap, as measured by luciferase light emission, measured in a chemiluminescence counter (cps).
   The cells were transfected with:
   a. pSV-SPORT-β_{c}/LepR-F3-ALK4CA + pSV-SPORT-GM-CSFRα/LepR-F3-Smad3 + pMG2-Smad4 + pXP2d2-rPAP1luci + pUT651.
   b. pSV-SPORT-β_{c}/LepR-F3-Smad3 + pSV-SPORT-GM-CSFRα/LepR-F3-ALK4CA + pMG2-Smad4 + pXP2d2-rPAP1luci + pUT651.
   c. pSV-SPORT-β_{c}/LepR-F3 + pSV-SPORT-GM-CSFRα/LepR-F3-Smad3 + pMG2-Smad4 + pXP2d2-rPAP1luci + pUT651.
   d. pSV-SPORT-β_{c}/LepR-F3-Smad3 + pSV-SPORT-GM-CSFRα/LepR-F3 + pMG2-Smad4 + pXP2d2-rPAP1luci + pUT651.
   e. pSV-SPORT-β_{c}/LepR-F3-ALK4CA + pSV-SPORT-GM-CSFRα/LepR-F3 + pMG2-Smad4 + pXP2d2-rPAP1luci + pUT651.
   f. pSV-SPORT-β_{c}/LepR-F3 + pSV-SPORT-GM-CSFRα/LepR-F3-ALK4CA + pMG2-Smad4 + pXP2d2-rPAP1luci + pUT651.
   g. pSV-SPORT-β_{c}/LepR-F3-ALK4CA + pSV-SPORT-GM-CSFRα/LepR-F3-Smad3 + pMG2 + pXP2d2-rPAP1luci + pUT651.
   h. pSV-SPORT-β_{c}/LepR-F3-Smad3 + pSV-SPORT-GM-CSFRα/LepR-F3-ALK4CA + pMG2 + pXP2d2-rPAP1luci + pUT651.
   i. pSV-SPORT-β_{c}/LepR-F3-ALK4CA + pSV-SPORT-GM-CSFRα/LepR-F3-Smad3 + pMET7-Smad4 + pXP2d2-rPAP1luci + pUT651.
   j. pSV-SPORT-β_{c}/LepR-F3-Smad3 + pSV-SPORT-GM-CSFRα/LepR-F3-ALK4CA + pMET7-Smad4 + pXP2d2-rPAP1luci + pUT651.

### Examples

### Materials and methods to the examples

### Cell lines, transfection and infection procedures

Transfections were performed according to the calcium phosphate method (Graham and van der Eb, 1973).

Recombinant mouse leptin, recombinant human leukemia inhibitory factor (LIF) and recombinant human erythropoietin (Epo) were all purchased from R&D Systems. Typical stimulation conditions were 100ng/ml leptin, 1 ng/ml LIF and 50 ng/ml Epo.

For production of ecotropic retrovirus harbouring the gp130-CIS or LacZ coding sequence, φNX-Eco cells were seeded at a density of 6x10⁶ cells/petridish the day prior to transfection. Cells were transfected with 50 µg of the retroviral vector pBG1-CIS according to the calcium phosphate procedure. 25 µM chloroquine was added 5 min. before transfection. Medium was harvested 24 and 48 hours post transfection, filtered over a 0.22 µm GV filter (Millipore) and stored at -80°C. Packaging of the HEK cDNA library was performed as described above with the exception that 1.6x10⁷ cells in a 175cm² falcon were transfected with 87 µg pBG1-HEK293cDNA. For titer determination, 10% pMFG-EGFP (gift from Dr. Mulligan, Cambridge, MA) was included in the DNA in a parallel experiment. The virus titer was approx. 5x10⁶ infectious units/ml as determined by FACS analysis of EGFP expressing cells.

For infection with CIS"prey", target cells were seeded at a density of 2x10⁴ cells/well in a 24-well plate, and 10⁶ in 75cm² culture flasks. The day after, cells were incubated for 24-48 hours with supernatant containing virus, diluted in medium as indicated. Polybrene (Sigma) was added at a final concentration of 2.5 µg/ml. After infection, cells were stimulated with Epo (50 ng/ml) for 24-48 hours, followed by puromycin (1-2 µg/ml as indicated; Sigma) selection for 10 days.

### Construction of bait, prey and reporter/selector constructs

### Generation of the EpoR/LepR chimera

All polymerase chain reactions (PCR) were performed using Pfu polymerase (Stratagene, typically 2,5-5U Pfu were used per reaction). The mouse leptin receptor (LepR) transmembrane and intracellular parts (amino acids 839-1162) were amplified by PCR using forward primer MBU-O-447 that contains a PacI restriction enzyme recognition site and the reverse primer MBU-O-448 that contains both a linker sequence (Gly-Gly-Ser) and a multi cloning site (MCS) with SalI, SacI, Spel, NotI and Xbal recognition sites. Only one amino acid from the extracellular part of the LepR was included in the fragment (Gly). Primer design resulted in the insertion of an Asn between the PacI generated Leu-Ile sequence and the extracellular Gly. The amplicon was gel-purified and ligated in the pCR®-Blunt vector (Invitrogen). PacI-SacI digestion on this pCR®-Blunt construct results, after gel-purification, in the desired LepR fragment.

The pSV-SPORT-EpoR/IFNaR2-2 (Pattyn *et al*, 1999). vector expresses an EpoR/IFNaR2-2 receptor chimera and was constructed as follows: RNA was isolated from 5x10⁶ TF-1 cells using the RNeasy kit (Qiagen). RT-PCR was performed as follows: 2 µl (2µg) of oligodT (12-18 mer; Pharmacia) was added and incubated at 70°C for 10 min., the reaction mixture was chilled on ice for 1 min., cDNA was prepared by adding 4 µl of 10x RT buffer (Life Sciences), 1µl 20 mM dNTP's (Pharmacia), 2µl 0,1M DTT, and 1µl of MMLV reverse transcriptase (200U; Superscript RT; Life Technologies) to an end volume of 20µl. Incubations were as follows: RT for 10 min., 42°C for 50 min., 90°C for 5 min., and 0°C for 10 min. Following this, 0,5 µl RnaseH (2U ; Life Technologies) was added and the mixture was incubated at 37°C for 20 min., followed by chilling on ice. PCR on this cDNA was performed using Pfu enzyme (5 U; Stratagene). Forward primer (MBU-O-167) and reverse primer (MBU-O-308) were designed to amplify the extracellular part of the EpoR (amino acids 1-249) between a Kpnl and PacI site. A band of correct size was purified and the DNA was digested with Kpnl and PacI and was inserted into the KpnI-PacI opened pSV-SPORT-IL-5Rα/IFNaR2-2 vector. This vector contains a chimeric receptor that has the extracellular domain of the IL-5Rα receptor, fused to the transmembrane and intracellular domains of IFNaR2-2. By site-specific mutagenesis, a PacI site was added to the fusion point by means of the Quikchange™ site-directed mutagenesis kit (Stratagene, La Jolla) which resulted in the insertion of two amino acids (Leu-Ile) before the most membrane-proximal, extracellular amino acid (Lys) of IFNaR2-2. Hence, using the KpnI site that precedes the coding sequence and the created PacI site on the extracellular/transmembrane domain fusion site, the extracellular domain of IL-5Rα could be exchanged by the one of EpoR, as described above.

The LepR fragment generated by PacI-SacI digestion was ligated in the PacI-SacI digested and gel-purified pSV-SPORT-EpoR/IFNaR2-2 vector, resulting in pSV-SPORT-EpoR/LepR.

### Generation of IL-3Rα/LepR, IL-5Rα/LepR, GM-CSFRα/LepR and β_{c}/LepR chimeras

The pSV-SPORT-IL-5Rα/IFNaR2-2 and pSV-SPORT-β_{c}/IFNaR1 vectors express an IL-5Rα/IFNaR2-2 and a β_{c}/IFNaR1 chimera, respectively, composed of the extracellular portion of the IL-5Rα or β_{c} chain, and the transmembrane and intracellular parts of the IFNaR2-2 or IFNaR1. A PacI site was used to generate the fusion site just preceding the transmembrane segment. The IFNaR2-2 or IFNaR1 parts in these vectors were replaced by the same segments of the LepR, using the PacI site and an Xbal site which is located just after the IFNaR2-2 or IFNaR1 stop codon. Therefore, the LepR fragment was generated by a PacI-XbaI digest of the pSV-SPORT-EpoR/LepR vector (see example 1), and was inserted into the PacI-XbaI opened and gel-purified pSV-SPORT-IL-5Rα/IFNaR2-2 and pSV-SPORT-β_{c}/IFNaR1 vectors, resulting in the vectors pSV-SPORT-IL-5Rα/LepR and pSV-SPORT-β_{c}/LepR.

The pSV-SPORT-IL-3Rα/LepR and pSV-SPORT-GM-CSFRα/LepR vectors were constructed as follows: the extracellular portion of the IL-3Rα and GM-CSFRα chains were amplified using standard RT-PCR procedures with Pfu polymerase. 2 µl TF-1 cDNA was used as input. Forward primers were MBU-O-752 (IL-3Rα) and MBU-O-754 (GM-CSFRα), and generated a Kpnl site. Reverse primers MBU-O-753 (IL-3Rα) and MBU-O-755 (GM-CSFRα), contain a PacI site allowing in frame fusion to the LepR.

After subcloning in the pCR®-Blunt vector, the KpnI-PacI excised extracellular fragments were ligated into the KpnI-PacI opened pSV-SPORT-IL-5Rα/LepR. For the GM-CSFRα construction, a partial KpnI digest was applied since the extracellular portion contained an intemal KpnI site. The resulting vectors, pSV-SPORT-IL-3Rα/LepR and pSV-SPORT-GM-CSFRα/LepR, contain chimeric receptors composed of the extracellular portion of the IL-3Rα or GM-CSFRα chain fused to the transmembrane and cytoplasmatic tail of the LepR.

### Generation of the EpoR/LepR- F3 chimera

The mutant leptin receptors (Eyckerman *et al*., 1999) Y985-1077F and Y985-1077-1138F (LepR-F3; previously called F-all) were generated using the Quikchange™ site-directed mutagenesis procedure using Pfu polymerase (Stratagene) on the pMET7-LepR template. Mutagenic oligonucleotides were MBU-O-157, MBU-O-158, MBU-O-159, MBU-O-160, MBU-O-161 and MBU-O-162. Each single mutation was coupled to a change in restriction cleavage and was confirmed by restriction and DNA sequence analysis. The double and triple mutants were created using a sequential approach. Signalling properties of the generated mutants were investigated at the gene induction level using the rPAP1-luci reporter construct (see below) and Northern blot analysis of induction of Metallothionein II gene transcripts. The double Y985-1077F mutant showed a higher stimulation of the relevant genes compared to the wild type LepR, which is probably due to loss of recruitment of a SH2-module containing tyrosine phosphatase such as SHP-1 or SHP-2. The triple Y985-1077-1138F (LepR-F3) showed almost complete loss of induction due to elimination of the Box3 or STAT-3 association motif. This results in a receptor which still allows phosphorylation and activation of the associated JAK2 kinase but which cannot deliver a stimulatory signal to the studied genes.

PCR amplification on this pMET7-LepR-F3 vector template using MBU-O-447 and MBU-O-448 as forward and reverse primers, respectively, resulted in a LepR-F3 amplicon spanning the transmembrane and intracellular domains of LepR-F3 (+1 extra Gly of the extracellular part, see above), which was subcloned in the pCR®-Blunt vector. PacI-SacI digestion of the resulting plasmid yielded a DNA fragment containing the LepR-F3 sequence, which was ligated into PacI-SacI digested and gel-purified pSV-SPORT-EpoR/IFNaR2-2 vector (see above). This resulted in the pSV-SPORT-EpoR/LepR-F3, which was renamed to pSEL1.

### Generation of the prey vector

Prey constructs were generated in the pMET7 vector, which contains a strong constitutive hybrid SRα promoter (Takebe *et al*., 1988).

Through site-directed mutagenesis (Quikchange™, Stratagene) a unique Apal site was introduced after the pMET7 promoter and before the unique EcoRI site in the pMET7mcs vector resulting in pMET7mcsA (primers MBU-O-567 and MBU-O-568).

The pMET7mcs vector is a modified version of pMET7 containing an expanded MCS by insertion of the extra unique BglII, EcoRV, BstEII, AgeI and Xhol restriction sites. PCR amplification on the pSVL-gp130 template using the forward primer MBU-O-586 and the reverse primer MBU-O-443 generated a DNA fragment encoding a 158 amino acid-long intracellular fragment of the human gp130 chain, which contains 4 STAT-3 association motifs (amino acids 761-918, the stopcodon was not co-amplified). The forward primer contains from 5' to 3' an Apal restriction site, a Kozak consensus sequence, a flag-tag encoding sequence (Met-Asp-Tyr-Lys-Asp-Asp-Asp-Asp-Lys-Ile), and a BglII restriction site. The reverse primer encodes an additional hinge sequence (Gly-Gly-Ser) and contains an EcoRI recognition site. Apal and EcoRI digestion of the PCR product (after subcloning in pCR®-Blunt) and of pMET7-mcsA, allowed us to ligate the gp130 fragment into the pMET7 vector, generating the pMET7-flag-gp130 construct.

SV40 largeT antigen (SVT) was amplified using a vector from the HybriZAP-2.1 Two-Hybrid cDNA synthesis kit (Stratagene, pSV40) as template. Primers MBU-O-445 and MBU-O-446 were used to generate a DNA fragment encoding 448 amino acid between residues 261 and 708. The N-terminal deletion eliminates the nuclear targeting signal in SVT. The forward primer contains an EcoRI recognition site that allows in-frame ligation to the gp130-hinge sequence. The reverse primer contains additional Nrul, Xhol, BglII, NotI and Xbal restriction sites and also encodes the stop codon after the SVT coding sequence. Subcloning in pCR®-Blunt, followed by recovery of the cleaved amplicon with EcoRI and Xbal, allowed ligation in the EcoRI-XbaI opened pMET7-flag-gp130 vector, yielding pMET7-flag-gp130-SVT, which was renamed to pMG1-SVT. Digestion with EcoRI-XhoI or EcoRI-NotI allows the insertion of model preys or of cDNA libraries into this vector. In these cases, the SVT fragment acts as a 'stuffer'.

### Construction of the p53-SVT interaction trap vectors

A DNA fragment encompassing murine p53 was amplified with MBU-O-450 and MBU-O-451 using the p53 control plasmid from the HybriZAP-2.1 Two-Hybrid cDNA synthesis kit (Stratagene) as template. The forward primer contains a SalI restriction site that allows in-frame coupling to the EpoR/LepR-F3 hinge construct. The reverse primer contains a STOP codon and a Xbal restriction site. The 243 amino acid-long p53 fragment (amino acids 73-315) contains the interaction site with SVT, but lacks the nuclear targeting signal and the oligomerisation domain. Subcloning over pCR®-Blunt, digestion with Sall-Xbal and gel-purification yielded a fragment that was ligated into Sall-Xbal cut and gel-purified pSEL1 vector, resulting in pSEL1-p53.

Generation of the pMG1-SVT vector is described above.

Amplification using MBU-O-695 and MBU-O-696 as forward and reverse primer respectively, and using pMG1-SVT as template resulted in a SVT fragment (amino acids 261-708 + stopcodon) between BglII and Xbal recognition sites. The BglII-XbaI digested and purified PCR fragment was ligated into a BglII-XbaI cut and gel-purified pMG1-SVT vector, resulting in pMET7-SVT.

### Construction of EpoR-CIS interaction trap vectors

RNA was prepared from 5x10⁶ TF-1 cells using the RNeasy kit (Qiagen), and eluted in 50 µl water from which 10 µl was used as input for RT-PCR. RT-PCR was performed using standard reaction conditions as described in section I .1.1. An intracellular fragment of the human EpoR (amino acids 370-453) was amplified from 4µl TF1 cDNA using MBU-O-675 and MBU-O-676 as forward and reverse primer respectively, with two consecutive PCR reactions and an intermediate gel-purifrcation. Sacl and Xbal recognition sites are present in the forward and reverse primers respectively. The reverse primer also encodes a stop codon. After gel-purification of the PCR amplicon band of the correct size, the fragment was subcloned in pCR®-Blunt, digested with SstI (which has the same recognition site as SacI) and Xbal, and ligated into SstI-XbaI digested and gel-purified pSEL1 vector, resulting in the pSEL1-EpoR construct.

Through site directed mutagenesis (Quikchange™, Stratagene) a Tyr to Phe mutation on position 426 in the human EpoR was introduced in the pSEL1-EpoR construct resulting in an inactive EpoR fragment. Forward primer MBU-O-717 and reverse primer MBU-O-718 were used for the PCR-based mutagenesis which also resulted in the insertion of an EcoRl enzyme recognition site. Introduction of the mutation was confirmed by restriction and DNA sequence analysis. The construct was named pSEL1-EpoRY-F.

The complete coding region for mouse Cytokine Inducible SH2-containing protein CIS (amino acids 2-257) was amplified using MBU-O-677 and MBU-O-678 as forward and reverse primer respectively. The forward primer contains an EcoRl recognition site and the reverse primer contains a Xbal recognition site and the stop codon. The amplified and gel-purified fragment was subcloned into the pCR®-Blunt vector. The insert was recovered by EcoRI and Xbal digestion and gel-purification and was cloned into an EcoRI-XbaI digested and gel-purified pMG1-SVT vector, leading to the pMG1-CIS vector.

### Construction of the IRS1-Vav interaction trap vectors

Through a mutagenesis approach (Quikchange™, Stratagene) 4 amino acids (P₁₁₃₇-Y-M-P₁₁₄₀) within the mutant leptin receptor Y985-1077F (pMET7 LepR Y985-1077F) were exchanged for a phosphotyrosine encoding region from human IRS1 (Insulin Receptor Substrate 1 ; S₈₉₂-P-G-E-Y-V-N-t-E-F₉₀₁). This removes the functional STAT3 association motif within the leptin receptor. Primers MBU-O-515 and MBU-O-516 were used for the PCR-based mutagenesis. This construct was named pMET7 LepR-IRS1.

Using standard RT-PCR procedures with Pfu polymerase full size human GRB2 (Growth Receptor Bound 2; aa 1-217) was amplified using 2 µl HepG2 cDNA as input, and using MBU-O-467 and MBU-O-468 as forward and reverse primer respectively. The forward primer contains an extra EcoRI site which allows in frame fusion to the gp130 chain in the pMG1 vector and the reverse primer contains an extra Xbal site after the stopcodon. After subcloning in the pCR®-Blunt vector, the EcoRI-XbaI excised fragment was ligated into the EcoRI-XbaI cut pMG1 vector, resulting in pMG1-GRB2. The SH2 domain of GRB2 (aa 60-158) was amplified in the same way using primers MBU-O-469 and MBU-O-470 as forward and reverse respectively. The forward primer contains an extra EcoRI recognition site which allows in frame fusion to the gp130 chain, and the reverse primer contains an extra stop codon and a Xbal enzyine recognition site. After subcloning of the PCR fragment in the pCR®-Blunt vector, the EcoRI-XbaI generated fragment was ligated in the EcoRI-XbaI cut pMG1 vector, resulting in the pMG1-GRB2S vector.

A fragment of human GRB2 comprising the C-terminal SH3 domain (aa 159-217) was amplified using the pMG1-GRB2 construct as template and MBU-O-770 and MBU-O-468 as forward and reverse primer respectively. MBU-O-770 allows in frame fusion to the flag tag by a BglII site, MBU-O-468 is described above. After subcloning this PCR fragment in the pCR®-Blunt vector, the GRB2 fragment was inserted in the pMG1 vector by a BglII-XbaI based exchange, resulting in the pMET7-GRB2SH3 vector.

A fragment of human Vav (VavS: aa 259-789) was amplified using Pfu polymerase from mRNA of the human TF1 cell line by standard RT-PCR techniques. Primers were MBU-O-737 and MBU-O-738 as forward and reverse respectively. MBU-O-737 contains an extra EcoRI allowing in frame fusion to gp130, and MBU-O-738 contains a Stop codon and a Xhol enzyme recognition site. The amplified fragment was subcloned in the pCR®-Blunt vector and ligated in the pMG1 vector through an EcoRI-Xhol based exchange. The VavS fragment was also amplified using forward primer MBU-O-771, reverse primer MBU-O-741 and pMG1-VavS as template. The forward primer contains a BamHI site, which allows in frame fusion to the flag tag, and the reverse primer contains a stop codon and a Xbal restriction site. The amplicon was subcloned in the pCR®-Blunt vector, and excised with BamHI and Xbal. The purified fragment was cloned in a BglII-XbaI cut pMG1 vector, resulting in the pMET7-VavS construct.

### Construction of the pGL3-rPAP1-luci and pSEAP-rPAP1 reporter constructs

Genomic DNA was isolated from the rat pheochromocytoma PC12 cell line using the DNAzoI procedure (Gibco BRL). Optimal primers for PCR amplification of the rat PAP1 promoter were selected using the "Oligo Primer 3" program (http://www-genome.wi.mit.cdu/cgi-bin/primer3.cgi). Forward and reverse primers were MBU-O-222 and MBU-O-223 respectively. Amplification was performed using Taq polymerase in 30 cycles: 2' at 94°C, 2' at 57°C, 2' at 72°C, followed by a 10' filling-in reaction at 72°C. Optimal MgCl₂ concentration was determined to be 6mM. The promoter fragment was cloned after polishing with Klenow polymerase in the pCR®-Blunt vector.

The promoter fragment was cut from this plasmid construct using a successive PstI digest, Klenow treatment to polish this end, and BamHI digest, resulting in a blunt-sticky fragment. The gel-purified fragment was cloned into a Smal-BglII opened and gel-purified pGL3 control vector (Promega). Digestion with SstI-SpeI restriction enzymes and gel-purification resulted in a fragment that was cloned into a Sstl-Nhel and purified pGL3 basic vector, resulting in the pGL3- rPAP1-luci construct. DNA sequencing revealed 10 nucleotides differing from the published sequence, but without affecting the leptin-dependent induction of this promoter segment.

The full-length rPAP1 promoter fragment was excised from pGL3 rPAP1-luci using partial digestion with Kpnl and Xhol and ligated into the Kpnl-Xhol opened pXP2d2 vector (gift from Prof. S. Nordeen), resulting in pXP2d2-rPAPl-luci. The coding sequence for puromycin was amplified using primers MBU-O-719 and MBU-O-720 on the pIRESpuro2 (Clontech) template. Combined XhoI-XbaI digestion allowed insertion in the pXP2d2-rPAP1-luci construct, resulting in pXP2d2-rPAP1-puro^{R}.

Digestion of the pGL3-rPAPl-luci construct using MluI and Xhol restriction enzymes resulted in a fragment spanning the full size rPAP1 promoter. This fragment was gel-purified and ligated into a MluI-XhoI cut and gel-purified pSEAP vector (TROPIX, Perkin Elmer) resulting in the pSEAP-rPAP1 construct. Functionality of this construct was assayed by transient co-transfection of pMET7 LepR and pSEAP-rPAP1 in PC12 cells using the Phospha-Light™ reporter assay kit from TROPIX (Perkin Elmer).

### Construction of the pcDNA5/FRT-EpoR and pBG1-SVT, pBG1-CIS and pBG1-ccdB vectors

Insertion of the EpoR-LR-F3-EpoR into the pcDNA5/FRT vector was obtained by re-amplifying the complete chimeric construct using MBU-O-167 and MBU-O-769 on the pSEL1-EpoR template, followed by subcloning using Kpnl and NotI sites. This construct was named pcDNA5/FRT-EpoR.

The SVT fragment was re-amplified from pMG1-SVT using forward primer MBU-O-766 and MBU-O-446. BamHI-NotI digestion allowed insertion in the pBMN-Z retroviral vector (gift from G. Nolan), resulting in vector pBG1-SVT. EcoRI-NotI based exchange of the SVT fragment for CIS (pMG1-CIS) resulted in the pBG1-CIS vector.

To allow counter-selection for vector self-ligation in case of insertion of cDNA libraries in the pBG1 vector, the E. coli control of cell death gene (ccdB) was amplified using primers MBU-O-835 and MBU-O-83 and template pENTRY11 (Life Technologies), and cloned in the pBG1-CIS vector by an EcoRI-NotI restriction-based insertion, resulting in the pBG1-ccdB vector.

### Construction of the bait-modifying enzyme, substrate-bait and prey chimeras.

### Generation of the pSV-SPORT-GM-CSFRα/LepR-F3 and pSV-SPORT-β_{c}/LepR-F3 chimeras

The LepR fragment in pSV-SPORT-GM-CSFRα/LepR and pSV-SPORT-β_{c}/LepR was replaced by the LepR-F3 fragment of pSEL1 (pSV-SPORT-EpoR/LepR-F3) using the PacI and NotI site. Therefore, the LepR-F3 fragment was generated by a PacI-NotI digest of the pSEL1 construct, gel-purified and inserted into the PacI-NotI opened and gel-purified pSV-SPORT-GM-CSFRα or pSV-SPORT-β_{c} vectors, resulting in the vectors pSV-SPORT-GM-CSFRα/LepR-F3 or pSV-SPORT-β_{c}/LepR-F3.

### Generation of the pSV-SPORT-GM-CSFRα/LepR-F3-modifying enzyme chimera and pSV-SPORT-β_{c}/LepR-F3-modifying enzyme chimera and generation of the pSV-SPORT-GM-CSFRα/LepR-F3-bait chimera and pSV-SPORT-β_{c}/LepR-F3-bait chimera

The pSV-SPORT-GM-CSFRα/LepR-F3 and pSV-SPORT-β_{c}/LepR-F3 vectors were digested with Sall, gel-purified and the ends were polished by Klenow fragment (Boehringer Mannheim). These blunt ended vectors were incubated with Alkaline Phosphatase (Boehringer Mannheim) to dephosphorylate the blunt ends. For the modifying enzyme a construct containing the mouse cytoplasmic tail of ALK4 with mutation T206D, resulting in a constitutive active kinase, in the vector pGBT9 was obtained from Prof. D. Huylebroeck. The mutated cytoplasmic tail of ALK4 was removed from the construct by an EcoRI-BamHI digestion, gel-purified and incubated with Klenow fragment to polish the ends. This insert was ligated in the opened pSV-SPORT-GM-CSFRα/LepR-F3 and pSV-SPORT-β_{c}/LepR-F3 vectors resulting in pSV-SPORT-GM-CSFRα/LepR-F3-ALK4CA and pSV-SPORT-β_{c}/LepR-F3-ALK4CA.

For the bait a construct containing a human cDNA encoding the entire Smad3 protein in vector pcdef was a kind gift of Prof. D. Huylebroeck. The Smad3 insert was removed with an EcoRI-XhoI digestion, gel-purified and the ends were polished by Klenow fragment (Boehringer Mannheim). This Smad3 insert was ligated into the opened pSV-SPORT-GM-CSFRα/LepR-F3 and pSV-SPORT-β_{c}/LepR-F3 vectors (described above) resulting in pSV-SPORT-GM-CSFRα/LepR-F3-Smad3 and pSV-SPORT-β_{c}/LepR-F3-Smad3.

### Generation of the pMG2-prey chimera

For construction of the pMG2 vector, the pMG1-SVT vector was digested with EcoRI and NotI, followed by incubation with Klenow fragment (Boehringer Mannheim) to polish the ends. This blunt ended vector was incubated with Alkaline Phosphatase (Boehringer Mannheim) to dephosphorylate the blunt ends. Cassette rfB of the Gateway Vector Conversion System (Life Technologies) was then ligated into the opened vector leading to the pMG1-gateway vector. A PCR reaction using primers MBU-O-1094 and MBU-O-1076 on the pMG1-SVT template was performed, resulting in a fragment that contains gateway recombination sites. This fragment also contains a part (amino acids 905 - 918) of the gp130 chain. The fragment was then cloned in the pMG1-gateway vector using a two-step gateway reaction (Lifetechnologies), resulting in pMG2-SVT, a prey construct with a total of 6 STAT recruitment sites. The pMG2-SVT construct was digested by EcoRI-XhoI and the vector was gel-purified. For the prey we obtained a construct from Prof. D. Huylebroeck containing a cDNA encoding almost the entire human Smad4 protein, only lacking the first 3 amino acids. The Smad4 insert was removed by an EcoRI-XhoI digestion, gel-purified and ligated into the opened pMG2 vector.

### Construction of the reporter cell lines

### Selection of the Hek293T PAP21 reporter cell line

The Blasticidin system (Invitrogen) was used to create a stable cell line with an endogenous pSEAP-rPAP1 reporter construct. Sensitivity to the toxic agent Blasticidin (Invitrogen) of Hek293T cells was estimated to be 3 µg/ml. 10⁶ cells were seeded in a petridish and transfected the day after seeding using the Calcium Phosphate Transfection System (Life Technologies) according to the manufacturers instructions. A total of 20 µg DNA was transfected (18 µg of pSEAP-rPAP1seap and 2 µg of pcDNA6/V5-HisA, which contains a Blasticidin resistance gene). After 48 hours the transfected cells were seeded in 96 well plates at 10 cells per well. After 24 hours, Blasticidin was added at a concentration of 3 µg/ml and cells were maintained under selective conditions for 3-4 weeks. The resulting single cell clones were screened by stimulation for 24 hours with 20 ng/ml hyper-IL6 (fusion protein of IL-6 with its specific receptor IL6-Rα; Fischer *et al*., 1997). Hek293T PAP21 was selected as the best responsive cell line.

### Generation of the HEK293-16 cell line

Flp-In-293 cells (Invitrogen) were stably transfected with a plasmid containing expression cassettes for the mouse ecotropic retroviral receptor (mEcoR) and for neomycin resistance. The pool of neomycin resistant cells (resistant to 400 µg/ml geneticin, Life Technologies) were supertransfected, at a ratio of 5:1 respectively, with the following two plasmids: i) a plasmid carrying the cDNA encoding the puromycin resistance marker (puromycin-N-acetyl-transferase) under control of promoter sequences of rPAP1 (pXP2d2-rPAP1-puro^{R}), ii) a plasmid carrying the cDNA for the blasticidin resistance marker (blasticidin S deaminase), under the control of the EM7 promoter (pcDNA6/V5-His, Invitrogen). After selection in blasticidin S (5 µg/ml, Invitrogen), single colonies were picked and seeded in 24 well plates. Puromycin resistance (1 µg/ml, Sigma; added 48 hours after seeding) was monitored in the absence or presence of LIF (1ng/ml). After another 5 days, surviving cells were stained with crystal violet using a standard procedure.

### RT-PCR analysis

Unless otherwise stated, cells were lysed in 100 µl RLT buffer (RNeasy® method, Qiagen) and chromosomal DNA was sheared using Qiashredder columns (Qiagen). Beads were pre-treated according to the manufacturers' instructions (Dynabeads M-280 Streptavidin, Dynal). Briefly, Dynabeads were washed twice in a high salt buffer (1M NaCl, 10mM Tris HCl pH7,5 and 1mM EDTA), and were incubated with 200 pmoles of biotinylated oligonucleotide directed against the gp130 chain (5' GGGCTGGGTAGACTCGGATCTTGAGAAGAC). Next, beads were washed three times in the above mentioned high salt buffer and resuspended in a low salt buffer (0,15M NaCl, 10mM Tris HCl pH7,5 and 1mM EDTA) to a concentration of 10 µg/µl. 5 µl of this suspension was added to 100 µl total lysate diluted 1/5 in the high salt buffer. 15' minutes after gentle rotation at room temperature, beads were washed three times with low salt buffer and eluted in 30 µl water for 2' at 65°C. 15 µl of this sample was used as input for a standard RT-PCR reaction with the Qiagen OneStep RT-PCR Kit. Primers 5' GGCATGGAGGCTGCGACTG and 5' TCGTCGACCACT GTGCTGGC were used for amplification of the "prey" fragment. In a pilot experiment using CIS as "prey" template, efficient amplification was obtained with lysate from less than 10³ cells.

### Reporter assays, binding assays, cell survival assay and FACS analysis

Luciferase was measured after lysis of the cells and addition of luciferase substrate (Luciferin, Duchefa). Light emission was measured using a TopCount chemiluminescence counter (Canberra Packard). All luciferase measurements were normalized using an expression construct constitutively expressing β-galactosidase (pUT651), which was measured in triplicate for every transfection using the GalactoStar kit (TROPIX).

Puromycin-resistant cell colonies were stained with crystal violet using a standard procedure.

Human erythropoietin receptor expression was monitored using goat anti-human EpoR polyclonal IgG (R&D Systems) at 2 µg/ml and Alexa488-conjugated donkey anti-goat IgG (Molecular Probes) at 4 µg/ml. For demonstration of the expression of the FLAG-tagged "prey" construct, cells were fixed and permeabilized with. Starfiqs™ according to the manufacturers' protocol (Immuno Quality Products) and were stained with an anti-FLAG mouse mAb (Sigma) at 8 µg/ml and fluorescein-conjugated sheep antimouse IgG (Amersharn), 1/50 diluted. All FACS analyses were performed on a FACSCalibur (Becton Dickinson).

### Generation of retroviral cDNA libraries and screening conditions

Construction of the HEK293 library was performed using standard procedures. Briefly, 5 µg of HEK293 polyA+mRNA was used as input for both oligo-dT and random primed first strand synthesis with Superscript II reverse transcriptase (Life Technologies). Both the oligo-dT and random primers contain a Notl site. After second strand synthesis, adaptors containing an EcoRI site were ligated. The cDNA was analyzed using agarose gel electrophoresis and fragments between 0.5 and 2.5 Kbp were cloned unidirectionally in the pBG1-ccdB vector opened with EcoRI-NotI.

For screening, a total of 6.10⁷ "bait" expressing cells were seeded at a density of 2.10⁶ per 175 cm² tissue culture flasks. 24 hours after seeding, cells were infected with the retroviral HEK293"prey" cDNA library (complexity of 2.10⁶) for another 24 hours. After infection, cells were stimulated for 6,5 hours with 50 ng/ml Epo, and puromycin was added at a final concentration of 2 µg/ml for 20 days. Single cell colonies were picked and analysed using a functional assay and RT-PCR sequencing.

### Immunoprecipitations and Western blot analysis

For demonstration of EpoR"bait" phosphorylation, we transiently transfected approximately 3.10⁶ HEK293T cells with plasmids encoding the EpoR"bait" or the EpoR"bait" Y402F mutant, and the CIS/SOCS-2"preys". Cleared lysates (in modified RIPA buffer: 50 mM TrisHCl pH8.0; 200 mM NaCl; 1% NP40; 0.5 % DOC; 0.05% SDS; 2 mM EDTA; 1mM Na₃VO₄; 1mM NaF; 20 mM β-glycerophosphate; Complete™ protease inhibitor cocktail [Roche]) of stimulated and unstimulated cells were incubated with 2 µg goat anti-human EpoR polyclonal IgG and Protein G Sepharose (Amersham Pharmacia Biotech). After precipitation, polyacrylamide gel electrophoresis and blotting, phosphorylation was revealed using PY20 antibody (Transduction Laboratories).

Immunoprecipitation of "preys" was performed after transfection of EpoR/EpoRF"baits" and the SOCS-2"prey"in HEK293T cells. Incubation of cleared lysates (modified RIPA) with anti-FLAG M2 affinity gel, allowed SOCS-2"prey" precipitation. Phosphorylation of the "prey" was revealed using the PY20 antibody. "Prey" expression levels were verified after stripping the blots and reprobing with anti-FLAG antibody.

STAT3 phosphorylation was demonstrated using the Phospho-STAT3 (Tyr705) Antibody (Cell Signaling) according to the manufacturers instructions. STAT3 expression was verified on the same blots using anti-STAT3 antibody (Transduction Laboratories).

### Example 1: Functionality of EpoR-LepR chimera in the Hek293T PAP21 cell line

To determine the functionality of the EpoR/LepR chimera, 3 combinations of plasmids were transfected into Hek293T PAP21 cells:
a. pSV-SPORT + pMET7mcs + pGL3-rPAP1-luci + pUT651
b. pSV-SPORT EpoR/LepR + pMET7mcs + pGL3-rPAP1-luci + pUT651
c. pMET7 LepRY985/1077F + pMET7mcs + pGL3-rPAP1-luci +pUT651

Transfection was performed according to the calcium phosphate method (Graham and van der Eb, 1973). A precipitate was formed using 3,4 µg as total DNA input (0.4 µg for pUT651, 1 µg for each of the others) into a 300 µl total mixture. 200 µl of this mixture was added to 4x10⁵ Hek293T PAP21 cells seeded the day before transfection into a six well plate (Falcon). 6 hours after adding the mixture, the cells were washed once with Dulbecco's PBS (Life Technologies) and new DMEM medium (Life Technologies) was added. Two days after transfection, medium was removed and cells were resuspended using 200 µl Cell Dissociation Agent (Life Technologies). After neutralization with 1200 µl DMEM medium, 50 µl of the cell suspension was seeded into a 96 well plate (Costar) in triplicate for every condition; and stimulated by adding recombinant human leptin (R&D systems) to a final concentration of 100 ng/ml, or recombinant human erythropoietin (R&D systems) to a final concentration of 0.5 units/ml, or the combination of leptin (same concentration as above) plus forskolin (Sigma, 10 µM final concentration), or the combination of erythropoietin (same concentration as above) plus forskolin (same concentration as above). A non-stimulated negative control was also incorporated in the experiment. Forskolin is a chemical agent that activates the adenylate cyclase that is present within the cells, which leads to heightened levels of the second messenger cAMP. Treatment of transfected cells with forskolin alone did not result in a significant induction of luciferase activity. Through an undefined mechanism, cAMP elevation leads to strong co-stimulation with the leptin signal on PAP1 induction (Eyckerman *et al.,* 1999). 24 hours after stimulation, the cells were lysed in the wells and luciferase substrate (Luciferin, Duchefa) was added. Light emission was measured using a TopCount chemiluminescence counter (Canberra Packard). The mock control transfection (transfection a) showed no signal in all cases. Results are shown in figure 2. The transfection with the EpoR/LepR chimera resulted in a 3,7 fold induction with erythropoietin, and a 6,5 fold induction with erythropoietin and forskolin. No significant signal was detected when stimulated with leptin, nor with leptin + forskolin. In the cells transfected with the LepR Y985/1077F mutant a 33,2 fold induction was detected when stimulated with leptin, and a 37,6 fold induction was detected when co-stimulated with forskolin. No signal was detected in both erythropoietin and erythropoietin + forskolin stimulated cells. All results were normalized using the intemal transfection control vector pUT651 and the GalactoStar kit (See above).

The difference in induction between EpoR/LepR and LepR Y985/1077F is very likely due to the elimination in the latter receptor construct of tyrosines involved in the recruitment of tyrosine phosphatases and SOCS proteins to the complex, leading to an enhanced signal (Eyckerman *et al*., 1999).

### Example 2: Functionality of p53-SV40 LargeT interaction trap

To investigate the functionality of this modification-independent interaction, the following plasmid combinations were transfected into 4x10⁵ Hek293T cells seeded in a 6 well plate the day before transfection:
a. pSV-SPORT + pMG1-SVT + pGL3-rPAP1-luci + pUT651
b. pSV-SPORT + pMG1-CIS + pGL3-rPAP1-luci + pUT651
c. pSV-SPORT + pMET7-SVT + pGL3-rPAP1-luci + pUT651
d. pSEL1-p53 + pMG1-SVT + pGL3-rPAP1-luci + pUT651
e. pSEL1-p53 + pMG1-CIS + pGL3-rPAP1-luci + pUT651
f. pSEL1-p53 + pMET7-SVT + pGL3-rPAP1-luci + pUT651

A 300 µl precipitation mixture was prepared which contained 3.1 µg DNA (0.1 µg of pUT651, 1 µg of each of the others). 200 µl was added to the cells for 6 hours after which they were washed once with Dulbecoo's PBS. After washing, DMEM medium was added to the cells. After 24 hours cells were resuspended using 200 µl Cell Dissociation Agent which was neutralized with 2200 µl DMEM medium. Of this cell suspension 40 µl was brought into a 96 well plate for each transfection and stimulation was performed in triplicate. 60 µl DMEM was added to an end volume of 100 µl and 24 hours later, cells were stimulated for 24 hours with erythropoietin or erythropoietin plus forskolin (same concentrations as described above). A non-stimulated negative control was also included in the experiment. Luciferase measurements are shown in figure 3.

Transfected cells from transfections a, b and c showed no significant induction of the reporter construct under all conditions tested. A 9,4 fold induction and a 14,6 fold induction was detected in transfected cells from transfection d, after stimulation with erythropoietin and erythropoietin plus forskolin respectively, implying an interaction-dependent signal. No signal was detected in transfection e and f. This implies a specific interaction, which leads to gp130-dependent STAT-3 activation. All results were normalized using the intemal transfection control vector pUT651 and the GalactoStar kit (See above).

### Example 3: Functionality of the EpoR-CIS phosphorylation-dependent interaction trap

To determine the functionality of the EpoR-CIS phosphorylation-dependent interaction trap, the following plasmid combinations were transfected in 4x10⁵ Hek293T cells, which were seeded the day before transfection:
a. pSV-SPORT + pMG1-CIS + pGL3-rPAP1-luci + pUT651
b. pSV-SPORT + pMG1-SVT + pGL3-rPAP1-luci + pUT651
c. pSV-SPORT + pEF-FLAG-I/mCIS + pGL3-rPAP1-luci + pUT651
d. pSEL1-EpoR + pMG1-CIS + pGL3-rPAP1-luci + pUT651
e. pSEL1-EpoR + pEF-FLAG-I/mCIS + pGL3-rPAP1-luci + pUT651
f. pSEL1-EpoRY-F + pMG1-CIS + pGL3-rPAP1-luci + pUT651
g. pSEL1-EpoR + pMG1-SVT + pGL3-rPAP1-luci + pUT651

A precipitation mixture of 300 µl was prepared which contained 3,1 µg DNA (0,1 µg of pUT651, 1 µg of each of the others). 200 µl of this mixture was applied to the cells.

After 6 hours, the cells were washed once with Dulbecco's PBS, and DMEM medium was added. After 48 hours the cells were resuspended using 250 µl Cell Dissociation Agent. After neutralization with 2200 µl DMEM medium, 100 µl of this cell suspension was brought into a 96 well plate (Costar). The cells were stimulated with erythropoietin or erythropoietin plus forskolin (for final concentrations, see above). A non-stimulated negative control was also included in the experiment. Luciferase expression was measured 24 hours after stimulation using a TopCount chemiluminescense counter (Canberra Packard). Transfected cells from transfections a, b, c, e, f and g showed no significant induction of luciferase activity. Transfected cells from transfection d showed a 6,2 fold and a 10,5 fold induction with erythropoietin or erythropoietin plus forskolin, respectively. This indicates an erythropoietin-dependent phosphorylation of the EpoR bait, resulting in interaction between the CIS protein and EpoR. Interaction leads to gp130 phosphorylation, STAT activation and thus signalling toward the rPAP1 promoter, leading to luciferase activity (Figure 4).

### Example 4: Functionality of the IRS1-GRB2-Vav indirect interaction trap

In order to investigate the IRS1-GRB2-Vav indirect interaction trap, following combinations of plasmids were transfected in 4x10⁵ Hek293T cells, seeded the day before transfection:
a. pMET7mcs + pMG1-CIS + pGL3-rPAP1-luci + pUT651
b. pMET7mcs + pMG1-GRB2S + pGL3-rPAP1-luci + pUT651
c. pMET7mcs + pMG1-VavS + pGL3-rPAP1-luci + pUT651
d. pMET7 LepR-IRS1 + pMG1-CIS + pGL3-rPAP1-luci + pUT651
e. pMET7 LepR-IRS1 + pMG1-GRB2S + pGL3-rPAP1-luci + pUT651
f. pMET7 LepR-IRS1 + pMG1-VavS + pGL3-rPAP1-luci + pUT651

A 300 µl precipitation mixture was prepared containing 3,05 µg DNA (0,05 µg for pUT651, 1 µg for others). 200 µl of this mixture was added to the cells for 16 hours. After transfection the cells were washed once with Dulbecco's PBS and DMEM (both from Gibco BRL) was added. After 48 hours the cells were resuspended using 200 µl of Cell Dissociation Agent (Gibco BRL) which was neutralized by 1,8 ml DMEM medium. 100 µl of cell suspension was seeded in a Costar 96 well plate and stimulated in a final volume of 200µl with final concentrations of 100 ng/ml leptin, 100 ng/ml leptin plus 10 µM forskolin, 10 µM forskolin, or were left unstimulated. 24 hours after stimulation, luciferase and galactosidase activity assays were performed as described above. From these results (Figure 5) we can conclude that the cells from transfections a, b, c, and d show no significant induction of the rPAP1 promoter. Cells from transfection e show a slight induction with leptin and a moderate induction when co-stimulated with forskolin (2,5 fold), suggesting a direct interaction between IRS-1 and GRB2S. Transfection experiment f shows a clear induction of luciferase activity with leptin (5,2 fold), which is more pronounced when co-stimulated with forskolin (12,0 fold). This indicates an interaction between IRS1 and Vav, which is probably mediated through endogenous GRB2.

To investigate the specificity and the involvement of GRB2 in the interaction, and to test if the signal is generated by recruitment of the gp130 chain, a number of control experiments were performed.

The following combinations of plasmids were tested in the same way as described above:
a. pMET7 LepR-IRS1 + 200 ng pMG1-VavS + pGL3-rPAP1-luci + pUT651
b. pMET7 LepR-IRS1 + 200 ng pMG1-VavS + 200 ng pMET7 GRB2SH3 + pGL3-rPAP1-luci + pUT651
c. pMET7 LepR-IRS1 + 200 ng pMG1-VavS + 1000 ng pMET7 GRB2SH3 + pGL3-rPAP1-luci + pUT651

0,05 µg of pUT651 DNA and 1 µg of pMET7 LepR-IRS1 and pGL3-rPAP1-luci were added to the 300 µl precipitation mixture. The results (Figure 6) are shown in fold induction. The results clearly show a dose dependent inhibition of rPAP1 induction when GRB2SH3 is overexpressed. Due to competition with endogenous GRB2 for Vav binding, recruitment of the gp130-VavS fusion protein to the complex is blocked, resulting in a dose dependent reduction of rPAP1 promoter activation. From this we can conclude that the specific GRB2-VavS interaction is required for induction of luciferase activity.

In order to investigate the critical role of gp130 recruitment in rPAP1 promoter induction, following combinations of plasmids were transfected as described above :
a. pMET7 LepR-IRS1 + 200 ng pMG1-VavS + pGL3-rPAP1-luci + pUT651
b. pMET7 LepR-IRS1 + 200 ng pMG1-VavS + 200 ng pMET7 VavS + pGL3-rPAP1-luci + pUT651
c. pMET7 LepR-IRS1 + 200 ng pMG1-VavS + 1000 ng pMET7 VavS + pGL3-rPAP1-luci + pUT651

The 300 µl precipitation mixture contained also 0,05 µg of pUT651 DNA and 1 µg of pMET7 LepR-IRS1 and pGL3-rPAP1-luci DNA. From the normalized results (Figure 7) we can conclude that gp130 in the gp130-VavS fusion construct is essential for PAP1 promoter induction since dose dependent competition with uncoupled VavS leads to a significant reduction in luciferase activity.

### Example 5: optimalization of the method for library screening

To permit easy interaction-dependent cDNA library screening, a selection system was developed as outlined in Figure 8. A HEK293 cell clone was used (i) containing a FRT integration cassette in a transcriptionally active locus (Flp-In-293 cell line, Invitrogen), (ii) stably expressing the murine ecotropic retroviral receptor EcoR, and (iii) with a stably integrated pXP2d2-rPAP1-puro^{R} selection cassette that directs STAT-regulated expression of the puromycin resistance gene. Clone HEK293-16 showed high sensitivity to puromycin (1 µg/ml), but acquired puromycin resistance upon LIF (Leukemia Inhibitory Factor)-induced activation of endogenous gp130 (Figure 9A). A model screening experiment involved the following successive steps: (i) EpoR"bait" expression was obtained after Flp recombinase-assisted integration of the pcDNAS/FRT-EpoR"bait" vector. Isogenic cells were selected by growth in hygromycin-containing medium (100 µg/ml, 10days), and FACS analysis using anti-EpoR antibodies indicated that almost the whole cell population showed homogeneous expression of the chimeric "bait" receptor (Figure 9B). (ii) Hygromycin-resistant cells were subsequently infected with CIS"prey"-expressing retrovirus for 24-48 hours. Retroviral gene transfer was chosen to attain expression from single integrants (Kitamura *et al*., 1995; Kojima and Kitamura, 1999). (iii) Cells were treated with Epo (50 ng/ml) for another 24-48 hours prior to puromycin selection. As shown in Figure 9C, colony formation was only observed in Epo-stimulated HEK293-16 cells co-expressing EpoR-"bait" and gp130-CIS"prey" proteins. FACS analysis with anti-FLAG antibody of permeabilized, puromycin resistant cells confirmed expression of the "prey" polypeptide (Figure 9D). Rapid identification of expressed "prey" transcripts was performed using a RT-PCR procedure. Taking advantage of the fact that all "prey" polypeptides are fused to human gp130, a biotinylated, gp130-specific primer was used to select "prey" transcripts directly from cell lysates using streptavidin-magnetobeads. After reverse transcription, selective PCR amplification of the "prey" insert was obtained using a gp130/3'LTR primer pair. DNA sequence analysis of such amplicons recovered from Epo-stimulated, puromycin-resistant cells showed that transcripts encoding the gp130-CIS "prey" were expressed as expected (Figure 9D, inset). Figure 9E shows the results of a "spiking" experiment where a complex retroviral HEK293 cDNA library was mixed with a dilution series of retrovirus expressing the gp130-CIS "prey". A dose-dependent recovery of cell clones was observed, only in the presence of ligand. RT-PCR cycle sequencing in a parallel experiment allowed identification of gp130-CIS"prey" expression in 19 out of 21 clones analysed.

### Example 6: Library screening with the MAPPIT system

A screening experiment with the EpoR"bait" using a retroviral HEK293 cDNA library (2.10⁶ independent clones) was performed. To favour single integrants, 6.10⁷ HEK293-16 cells expressing the EpoR"bait" were infected with an estimated infection efficiency of 4 %. Three weeks after Epo stimulation and selection in medium containing 2 µg/ml puromycin, 33 colonies were picked and analysed in a functional assay (Figure 9F). Since all clones stably co-express "bait" and "prey", we were able to rapidly demonstrate specific interaction with the EpoR"bait" by co-transfection with the LR-F3, lacking the "bait", and the rPAP1-luciferase constructs. Three clones showed induction by Epo but not by leptin, indicating that the interaction occurred specifically with the Y402 EpoR motif. In one of these clones, RT-PCR analysis showed the presence of a specific 1700 bases amplicon and cycle sequencing revealed this fragment to encode SOCS-2, another member of the SOCS family. The latter was fused in frame within its pre-SH2 domain to gp130 (figure 9F). This again underscores the low background observed with this two-hybrid procedure. After subcloning in a plasmid vector, ligand-dependent phosphorylation of SOCS-2"prey" and of STAT3 was shown to depend on the phosphorylation of the Y402 EpoR motif, (Fig 9G). This demonstrates each of the phosphorylation (and interaction) steps preceding reporter gene activation.

### Example 7: the use of MAPPIT with heterodimeric receptors: Functionality of IL3R-, IL5R- and GM-CSFR-LepR chimeras in the Hek 293T cell line

In order to compare the functionality of the Epo, IL-3R, IL-5R and GM-CSFR LepR-chimera, following combinations of plasmids were transfected into 4x10⁵ Hek293T cells, seeded the day before transfection:
a. pSV-SPORT-EpoR/LepR + pGL3-rPAP1-luci + pUT651
b. pSV-SPORT-IL-3Rα/LepR + pSV-SPORT-β_{c}/LepR + pGL3-rPAP1-luci + pUT651
c. pSV-SPORT-IL-5Rα/LepR + pSV-SPORT-β_{c}/LepR + pGL3-rPAP1-luci + pUT651
d. pSV-SPORT-GM-CSFRα/LepR + pSV-SPORT-β_{c}/LepR + pGL3-rPAP1-luci + pUT651
e. pSV-SPORT-IL-3Rα/LepR + pGL3-rPAP1-luci + pUT651
f. pSV-SPORT-IL-5Rα/LepR + pGL3-rPAP1-tuci + pUT651
g. pSV-SPORT-GM-CSFRα/LepR + pGL3-rPAP1-luci + pUT651
h. pSV-SPORT-β_{c}/LepR + pGL3-rPAP1-luci + pUT651

A 300 µl precipitation mixture was prepared as described before, and which contained 0.05 µg of pUT651, and 1 µg of each of the others vectors. 200 µl of this mixture was applied to the cells. After 6 hours, the cells were washed once with Dulbecco's PBS. After 2 days, cells were resuspended using Cell Dissociation Agent, transferred to a 96 well plate (Costar). Transfections a - g were stimulated with 10000, 1000, 100, 10 pg/ml of the respective cytokine. Cells of transfection h, expressing only the pSV-SPORT-β_{c}/LepR, were treated with either 10 ng/ml Epo, IL-3, IL-5 or GM-CSF. A non-stimulated negative control was also included in the experiment. Luciferase expression was measured 24 hours after stimulation. Results are given in figure 10. The EpoR/LepR chimera and a combination of IL-3Rα/LepR with β_{c}/LepR have similar fold inductions. A signal above background is observed at cytokine concentrations of 1 ng/ml. The biological activity of IL-5 on cells transfected with the chimera IL-5Rα/LepR and β_{c}/LepR is less than these for IL-3 or Epo. Cells transfected with chimeras of the GM-CSFR and the LepR are much more sensitive to stimulation, with a clear 7.7 fold induction at a concentration as low as 10 pg/ml. The cells from the negative controls, transfections e, f, g and h, showed no significant induction of luciferase activity.

### Example 8: Functionality of the Smad3-Smad4 phosphorylation-dependent interaction trap

To determine the functionality of the Smad3-Smad4 phosphorylation-dependent interaction trap, the following plasmid combinations were transfected in 4x10⁵ Hek293T cells, which were seeded the day before transfection:
a. pSV-SPORT-β_{c}/LepR-F3-ALK4CA + pSV-SPORT-GM-CSFRα/LepR-F3-Smad3 + pMG2-Smad4 + pXP2d2-rPAP1-luci + pUT651.
b. pSV-SPORT-β_{c}/LepR-F3-Smad3 + pSV-SPORT-GM-CSFRα/LepR-F3-ALK4CA + pMG2-Smad4 + pXP2d2-rPAP1-luci + pUT651.
c. pSV-SPORT-β_{c}/LepR-F3 + pSV-SPORT-GM-CSFRα/LepR-F3-Smad3 + pMG2-Smad4 + pXP2d2-rPAP1-luci + pUT651.
d. pSV-SPORT-β_{c}/LepR-F3-Smad3 + pSV-SPORT-GM-CSFRα/LepR-F3 + pMG2-Smad4 + pXP2d2-rPAP1-luci + pUT651.
e. pSV-SPORT-β_{c}/LepR-F3-ALK4CA + pSV-SPORT-GM-CSFRα/LepR-F3 + pMG2-Smad4 + pXP2d2-rPAP1-luci + pUT651.
f. pSV-SPORT-β_{c}/LepR-F3 + pSV-SPORT-GM-CSFRα/LepR-F3-ALK4CA + pMG2-Smad4 + pXP2d2-rPAP1-luci + pUT651.
g. pSV-SPORT-β_{c}/LepR-F3-ALK4CA + pSV-SPORT-GM-CSFRα/LepR-F3-Smad3 + pMG2 + pXP2d2-rPAP1-luci + pUT651.
h. pSV-SPORT-β_{c}/LepR-F3-Smad3 + pSV-SPORT-GM-CSFRα/LepR-F3-ALK4CA + pMG2 + pXP2d2-rPAP1-luci + pUT651.
i. pSV-SPORT-β_{c}/LepR-F3-ALK4CA + pSV-SPORT-GM-CSFRα/LepR-F3-Smad3 + pMET7-Smad4 + pXP2d2-rPAP1-luci + pUT651.
j. pSV-SPORT-β_{c}/LepR-F3-Smad3 + pSV-SPORT-GM-CSFRα/LepR-F3-ALK4CA + pMET7-Smad4 + pXP2d2-rPAP1-tuci + pUT651.

A precipitation mixture of 300 µl was prepared which contained 2.92µg (0.02 µg for pUT651, 0.2 µg for pXP2d2-rPAP1-luci + 0.9 µg of each of the others). 200 µl of this mixture was applied to the cells. After 6 hours, the cells were washed once with Dulbecco's PBS, and DMEM medium was added. After 48 hours the cells were resuspended using 200 µl Cell Dissociation Agent. After neutralization with 2200µl DMEM medium, 40 µl of this cell suspension was brought into a 96 well plate (Costar).

The cells were stimulated in a final volume of 100 µl with final concentrations of 1 ng/ml GM-CSF, 1 ng/ml GM-CSF plus 10 µM forskolin, 10 µM forskolin or were left unstimulated. 24 hours after stimulation, luciferase and galactosidase activity assays were performed as described above. From these results (Figure 11) we can conclude that the cells from transfection c, d, e, f, g, h, i, and j showed no significant induction of luciferase activity. Cells from transfection a show a slight induction with GM-CSF (3 fold) which is increased to 37 fold when co-stimulation with forskolin. Transfection experiment b shows a clear induction of the rPAP1 promoter with GM-CSF (9 fold), which is again more pronounced when co-stimulated with forskolin (71 fold). This indicates a bait-modifying enzyme activity ALK4CA-dependent phosphorylation of the Smad3 bait, resulting in an interaction between Smad4 and phosphorylated Smad3. Interaction leads to gp130 phosphorylation, STAT activation and thus signalling towards the rPAP1 promoter, leading to luciferase activity.

### Example 9: the use op MAPPIT to screen compound-compound interactions comprising non-polypeptide compounds

An experiment is performed with Fujisporin, whereby FK506 is chemically linked to cyclosporin A (e.g. WO 94/18317). Altematively, bivalent compounds are obtained by fusing FK506 to tetracycline or to a steroid ligand. Such hybrid compounds have the capacity to interact with both protein partners: FKBP12 and cyclophilin (or tetracycline/steroid receptors).

Cells stably expressing the receptor/FKBP12 chimera are treated with the membrane-permeable divalent compound, and, after washing of excess compound, are infected or transfected to enforce "prey" expression. Altematively, both receptor/FKBP12 and "prey" chimeras are expressed simultaneously prior to addition of the compound. Careful compound dose-response experiments are performed; given the dimerizer effect of the hybrid compounds, bell-shaped dose-response curves are obtained. Addition of excess monovalent compound is used as specificity control and reduces significantly signal output.

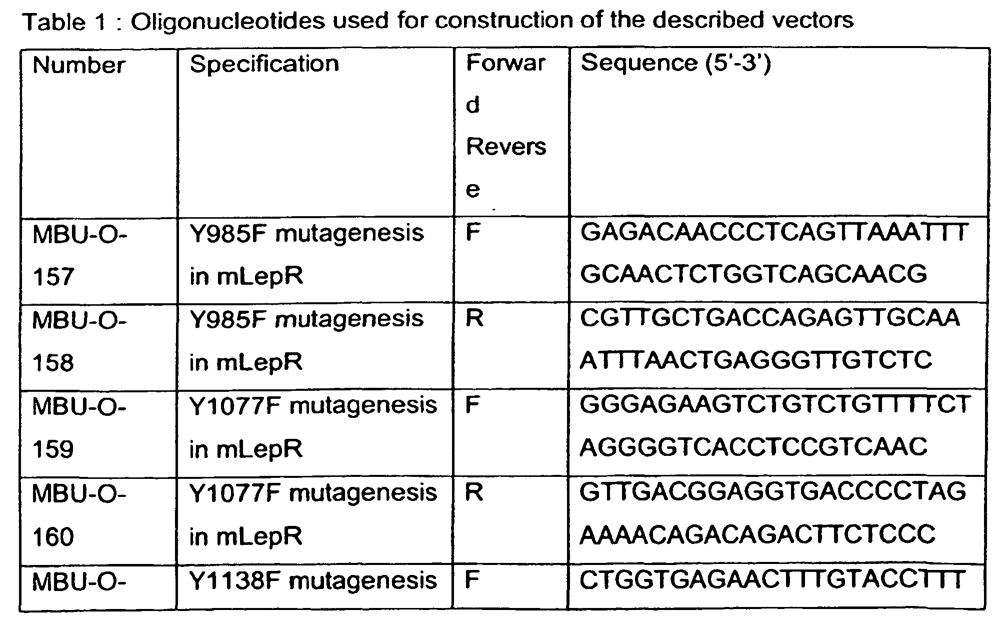

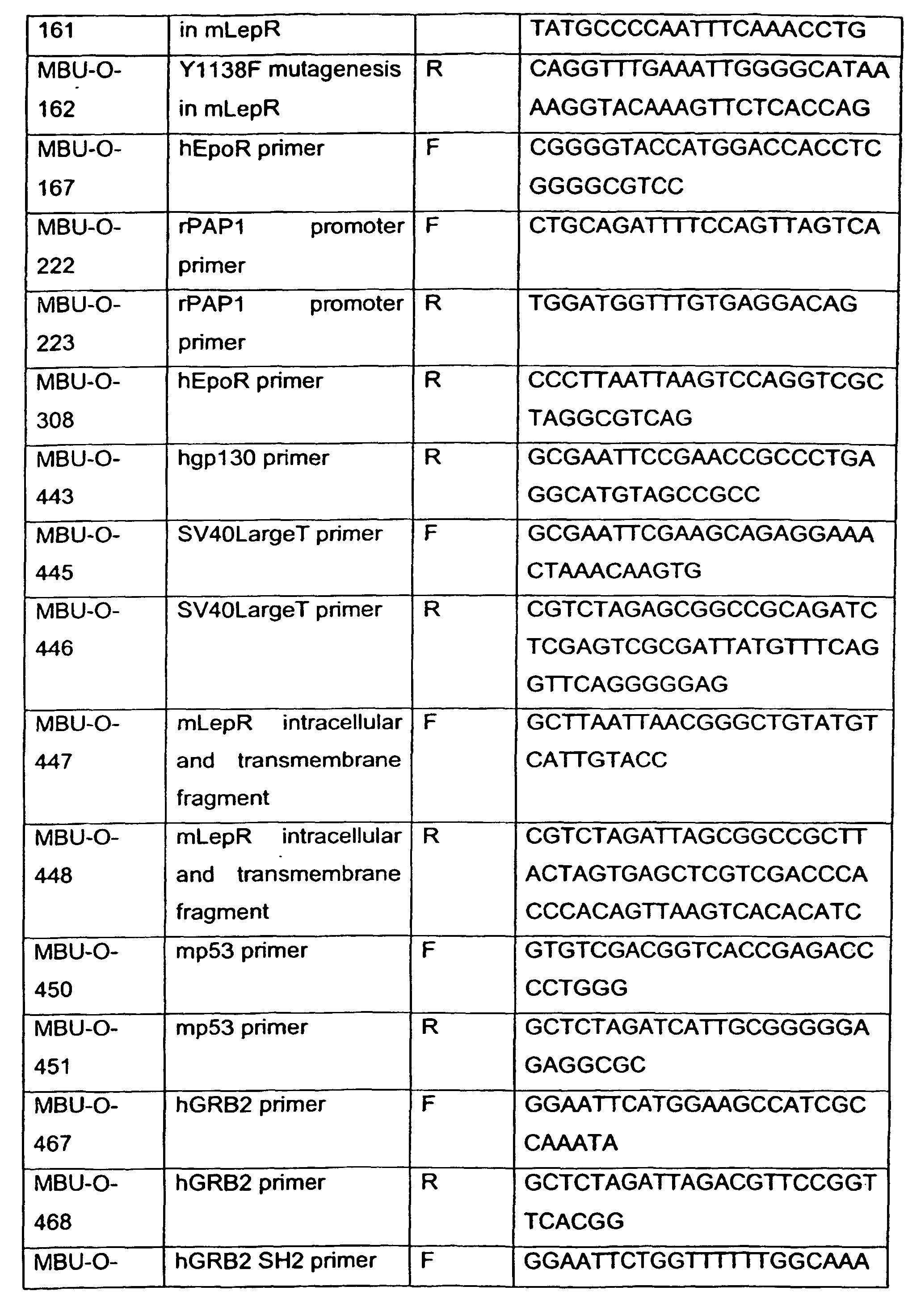

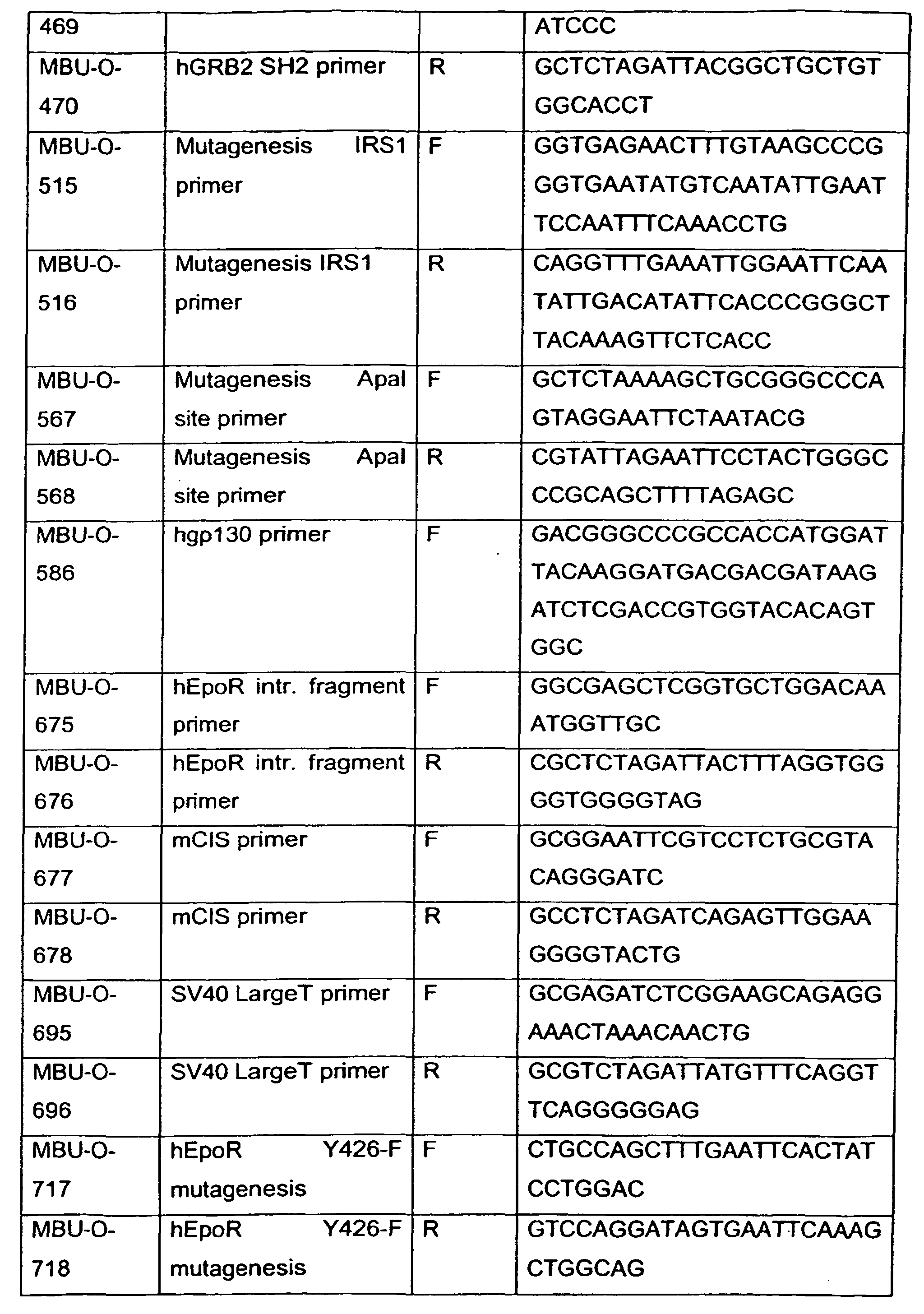

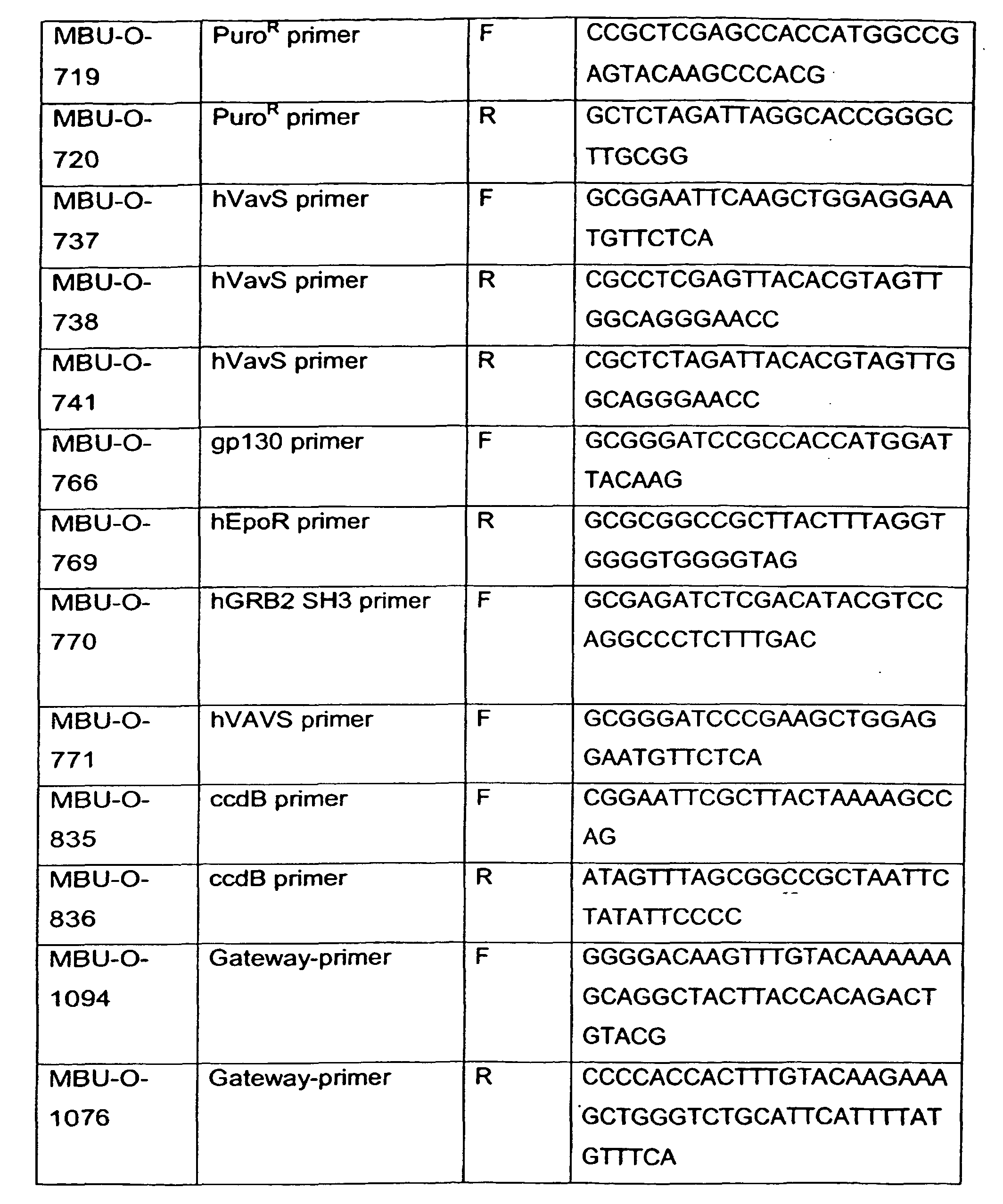

### References

- Eyckerman, S., Waelput, W., Verhee, A., Broekaert, D., Vandekerckhove, J. And Tavemier, J. (1999). Analysis of Tyr to Phe and fa/fa leptin receptor mutations in the PC12 cell line. *Eur. Cytokine Netw.,* 10, 549 - 556.
- Fields, S. and Song, O.K. (1989). A novel genetic system to detect protein-protein interactions. *Nature,* 340, 245 - 246.
- Fisher, M., Goldschinitt, J., Peschel, C., Kallen, K.J., Brakenhoff, J.P.J., Wollmer, A., Grötzinger, J. and Rose-John, S. (1997). A designer cytokine with high activity on human hemapoietic progenitor cells. *Nat. Biotechnol.,* 15, 142 - 145.
- Graham,F.L. and van der Eb, A.J. (1973). Transformation of rat cells by DNA of human adenovirus 5. *Virology,* 54, 536 - 539.
- Pattyn, E., Van Ostade, X., Schauvliege, L., Verhee, A., Kalai, M., Vandekerckhove, J. And Tavemier, J. (1999). Dimerization of the interferon type I receptor IFNaR2-2 is suffieicent for induction of interferon effector genes but not for full antiviral activity. *J. Biol. Chem.,* 274, 34838 - 34845.
- Kitamura, T., Onishi, M., Kinoshita, S., Shibuya, A., Miyajima, A. and Nolan, G.P. (1995) Efficient screening of retroviral cDNA expression libraries. *Proc. Natl. Acad. Sci. USA,* 14, 9146 - 9150.
- Kojima, T and Kitamura, T. (1999). A signal sequence trap based on a constituvely active cytokine receptor. *Nat. Biotechnol.,* 17, 487 - 490.
- Takebe, Y., Seiki, M., Fujisawa, J., Hoy, P., Yokota, K., Arai, K., Yoshida, M. and Arai, N. (1988) SR alph promoter: an efficient and versatile mammalian cDNA expression system composed of the simian virus 40 early promoter and the R-U5 segment of human T-cell leukemia virus type 1 log terminal repeat. *Mol.Cell.Biol*., 8, 466 - 472.
- Wiley, J.C., Wailes, L.A., Idzerda, R.L. and McKnight, G.S. (1999). Role of regulatory subunits and protein kinase inhibitor (PKI) in determining nuclear localization and activity of the catalytic subunit of protein kinase A. *J. Biol. Chem.,* 274, 6381 - 6387.

## Claims

1. A recombinant receptor, comprising an extracellular ligand binding domain and a cytoplasmic domain derived from a receptor, said cytoplasmic domain comprising a first interacting polypeptide, heterologous to said cytoplasmic domain, whereby said recombinant receptor is activated by binding of a ligand to said ligand binding domain and by binding of a second interacting polypeptide with the first interacting polypeptide.

2. A recombinant receptor according to claim 1 whereby said recombinant receptor is a homomultimerizing receptor.

3. A recombinant receptor according to claim 1 whereby said recombinant receptor is a heteromultimerizing receptor.

4. A recombinant receptor according to any of the claims 1-3 whereby the binding of said second interacting polypeptide is depending upon the modification state of said heterologous first interacting polypeptide.

5. A recombinant receptor according to claim 4 whereby said modification state is presence or absence of phosphorylation, acetylation, acylation, methylation, ubiquitinilation or glycosylation.

6. A recombinant receptor according to claim 4 whereby said modification state is occurrence of proteolytic cleavage or not.

7. A recombinant receptor according to any of the claims 4-6 whereby the change of said modification state is dependent upon binding of a ligand to the ligand-binding domain.

8. A recombinant receptor according to any of the claims 1-7 whereby said second interacting polypeptide is a fusion protein comprising a polypeptide which comprises at least one activation site of a receptor.

9. A recombinant receptor according to any of the preceding claims, whereby the cytoplasmic domain comprises the leptin receptor cytoplasmic domain of which at least one of the tyrosine phosphorylation sites has been inactivated, or a functional fragment of said inactivated leptin receptor cytoplasmic domain.

10. A vector encoding a recombinant receptor according to any of the claims 1-9.

11. An eukaryotic cell comprising a recombinant receptor according to any of the claims 1-9.

12. An eukaryotic cell according to claim 11, where said cell is a mammalian cell, a fungal cell or a plant cell.

13. A kit, comprising a cloning vector allowing the construction of a vector according to claim 10.

14. In vitro method to detect compound-compound binding using a recombinant receptor according to any of the claims 1-9.

15. Method to detect compound-compound binding according to claim 14, whereby said binding is modification state dependent.

16. Method to detect compound-compound binding according to claim 15, whereby said modification is phosphorylation, acetylation, acylation, methylation, ubiquitinilation or glycosylation.

17. Method to detect compound-compound binding according to any of the claims 14-16, whereby said binding is mediated by three or more partners.

18. Method to detect compound-compound binding according to claim 17, whereby one or more of the partners is not or not completely of proteineous nature.

19. A system to screen protein-protein interactions, comprising 1) a recombinant receptor, comprising an extracellular ligand binding domain and a cytoplasmic domain derived from a receptor, said cytoplasmic domain comprising a first interacting polypeptide heterologous to said cytoplasmic domain, 2) a fusion polypeptide, comprising a second interacting polypeptide and a polypeptide comprising at least one activation site of a receptor, and whereby said recombinant receptor is activated by binding of a ligand to said ligand binding domain and by binding of the first interacting polypeptide to the second interacting polypeptide.

## Patentansprüche

1. Rekombinanter Rezeptor, umfassend eine extrazelluläre Ligandenbindedomäne und eine von einem Rezeptor abstammende zytoplasmatische Domäne, wobei die zytoplasmatische Domäne ein erstes wechselwirkendes Polypeptid umfasst, das zu der zytoplasmatischen Domäne heterolog ist, wobei der rekombinante Rezeptor durch die Bindung eines Liganden an die Ligandenbindedomäne und durch die Bindung eines zweiten wechselwirkenden Polypeptids mit dem ersten wechselwirkenden Polypeptid aktiviert wird.

2. Rekombinanter Rezeptor gemäß Anspruch 1, wobei der rekombinante Rezeptor ein homomultimerisierender Rezeptor ist.

3. Rekombinanter Rezeptor gemäß Anspruch 1, wobei der rekombinante Rezeptor ein heteromultimerisierender Rezeptor ist.

4. Rekombinanter Rezeptor gemäß einem der Ansprüche 1-3, wobei die Bindung des zweiten wechselwirkenden Polypeptids von dem Modifikationszustand des heterologen ersten wechselwirkenden Polypeptids abhängt.

5. Rekombinanter Rezeptor gemäß Anspruch 4, wobei der Modifikationszustand in der An- oder Abwesenheit von Phosphorylierung, Acetylierung, Acylierung, Methylierung, Ubiquitinierung oder Glykosylierung besteht.

6. Rekombinanter Rezeptor gemäß Anspruch 4, wobei der Modifikationszustand im Auftreten oder Unterbleiben einer proteolytischen Spaltung besteht.

7. Rekombinanter Rezeptor gemäß einem der Ansprüche 4-6, wobei die Änderung des Modifikationszustands von der Bindung eines Liganden an die Ligandenbindedomäne abhängt.

8. Rekombinanter Rezeptor gemäß einem der Ansprüche 1-7, wobei das zweite wechselwirkende Polypeptid ein Fusionsprotein ist, umfassend ein Polypeptid, das wenigstens eine Aktivierungsstelle eines Rezeptors umfasst.

9. Rekombinanter Rezeptor gemäß einem der vorhergehenden Ansprüche, wobei die zytoplasmatische Domäne die zytoplasmatische Leptinrezeptordomäne, in welcher wenigstens eine der Tyrosin-Phosphorylierungsstellen inaktiviert wurde, oder ein funktionales Fragment der inaktivierten zytoplasmatischen Leptinrezeptordomäne, umfasst.

10. Vektor, kodierend einen rekombinanten Rezeptor gemäß einem der Ansprüche 1-9.

11. Eukaryotische Zelle, umfassend einen rekombinanten Rezeptor gemäß einem der Ansprüche 1-9.

12. Eukaryotische Zelle gemäß Anspruch 11, wobei die Zelle eine Säugetierzelle, eine Pilzzelle oder eine Pflanzenzelle ist.

13. Kit, umfassend einen Klonierungsvektor, der die Konstruktion eines Vektors gemäß Anspruch 10 erlaubt.

14. *In vitro*-Verfahren zum Nachweis einer Bindung Verbindung/Verbindung unter Verwendung eines rekombinanten Rezeptors gemäß einem der Ansprüche 1-9.

15. Verfahren zum Nachweis einer Bindung Verbindung/Verbindung gemäß Anspruch 14, wobei die Bindung vom Modifikationszustand abhängig ist.

16. Verfahren zum Nachweis einer Bindung Verbindung/Verbindung gemäß Anspruch 15, wobei die Modifikation Phosphorylierung, Acetylierung, Acylierung, Methylierung, Ubiquitinierung oder Glykosylierung ist.

17. Verfahren zum Nachweis einer Bindung Verbindung/Verbindung gemäß einem der Ansprüche 14-16, wobei die Bindung durch drei oder mehr Partner vermittelt wird.

18. Verfahren zum Nachweis einer Bindung Verbindung/Verbindung gemäß Anspruch 17, wobei wenigstens einer der Partner nicht oder nicht vollständig proteinöser Natur ist.

19. System zur Durchmusterung von Protein-Protein-Wechselwirkungen, wobei das System Folgendes umfasst:
1) einen rekombinanten Rezeptor, umfassend eine extrazelluläre Ligandenbindedomäne und eine von einem Rezeptor abstammende zytoplasmatische Domäne, wobei die zytoplasmatische Domäne ein erstes wechselwirkendes Polypeptid umfasst, das zu der zytoplasmatischen Domäne heterolog ist,
2) ein Fusionspolypeptid, umfassend ein zweites wechselwirkendes Polypeptid und ein Polypeptid, umfassend wenigstens eine Aktivierungsstelle eines Rezeptors,
und wobei der rekombinante Rezeptor durch die Bindung eines Liganden an die Ligandenbindedomäne und durch die Bindung des ersten wechselwirkenden Polypeptids an das zweite wechselwirkende Polypeptid aktiviert wird.

## Revendications

1. Récepteur recombinant, comprenant un domaine extracellulaire de fixation du ligand et un domaine cytoplasmique dérivé d'un récepteur, ledit domaine cytoplasmique comprenant un premier polypeptide d'interaction, hétérologue par rapport audit domaine cytoplasmique, de sorte que ledit récepteur recombinant est activé par la fixation d'un ligand sur ledit domaine de fixation du ligand et par la fixation d'un second polypeptide d'interaction au premier polypeptide d'interaction.

2. Récepteur recombinant selon la revendication 1 dans lequel ledit récepteur recombinant est un récepteur d'homomultimérisation.

3. Récepteur recombinant selon la revendication 1 dans lequel ledit récepteur recombinant est un récepteur d'hétéromultimérisation.

4. Récepteur recombinant selon l'une quelconque des revendications 1-3 dans lequel la fixation dudit second polypeptide d'interaction est dépendante de l'état de modification dudit premier polypeptide d'interaction hétérologue.

5. Récepteur recombinant selon la revendication 4 dans lequel ledit état de modification est une présence ou une absence de phosphorylation, d'acétylation, d'acylation, de méthylation, d'ubiquitinylation ou de glycosylation.

6. Récepteur recombinant selon la revendication 4 dans lequel ledit état de modification est l'apparition ou non d'un clivage protéolytique.

7. Récepteur recombinant selon l'une quelconque des revendications 4-6 dans lequel le changement dudit état de modification est dépendant de la fixation d'un ligand sur le domaine de fixation du ligand.

8. Récepteur recombinant selon l'une quelconque des revendications 1-7 dans lequel ledit second polypeptide d'interaction est une protéine de fusion comprenant un polypeptide qui comprend au moins un site d'activation d'un récepteur.

9. Récepteur recombinant selon l'une quelconque des revendications précédentes, dans lequel le domaine cytoplasmique comprend le domaine cytoplasmique du récepteur de la leptine dans lequel au moins un des sites de phosphorylation de la tyrosine a été inactivé, ou un fragment fonctionnel dudit domaine cytoplasmique du récepteur de la leptine inactivé.

10. Vecteur codant pour un récepteur recombinant selon l'une quelconque des revendications 1-9.

11. Cellule eucaryote comprenant un récepteur recombinant selon l'une quelconque des revendications 1-9.

12. Cellule eucaryote selon la revendication 11, où ladite cellule est une cellule de mammifère, une cellule fongique ou une cellule végétale.

13. Kit, comprenant un vecteur de clonage permettant la construction d'un vecteur selon la revendication 10.

14. Procédé in vitro pour détecter une association composé-composé à l'aide d'un récepteur recombinant selon l'une quelconque des revendications 1-9.

15. Procédé pour détecter une association composé-composé selon la revendication 14, dans lequel ladite association est fonction de l'état de modification.

16. Procédé pour détecter une association composé-composé selon la revendication 15, dans lequel ladite modification est une phosphorylation, une acétylation, une acylation, une méthylation, une ubiquitinylation ou une glycosylation.

17. Procédé pour détecter une association composé-composé selon l'une quelconque des revendications 14-16, dans lequel ladite association est réalisée via trois partenaires ou plus.

18. Procédé pour détecter une association composé-composé selon la revendication 17, dans lequel un ou plusieurs des partenaires n'est pas ou n'est pas complètement de nature protéinique.

19. Système de criblage d'interactions protéine-protéine, qui comprend 1) un récepteur recombinant, comprenant un domaine extracellulaire de fixation du ligand et un domaine cytoplasmique dérivé d'un récepteur, ledit domaine cytoplasmique comprenant un premier polypeptide d'interaction hétérologue par rapport audit domaine cytoplasmique, 2) un polypeptide de fusion, comprenant un second polypeptide d'interaction et un polypeptide comprenant au moins un site d'activation d'un récepteur, et dans lequel ledit récepteur recombinant est activé par la fixation d'un ligand sur ledit domaine de fixation du ligand et par la fixation du premier polypeptide d'interaction au second polypeptide d'interaction.
